# EUROPÄISCHE PATENTSCHRIFT

(11) **EP 3 082 988 B1**
(45) Veröffentlichungstag und Bekanntmachung des Hinweises auf die Patenterteilung: **27.03.2019**
(21) Anmeldenummer: 14821589.0
(22) Anmeldetag: 17.12.2014
(51) Int. Cl.: B01D 11/04, B01D 3/14, B01D 3/40, C07C 51/48, B01D 11/00

(54) **EXTRAKTIONSKOLONNE UND VERFAHREN ZUM EXTRAHIEREN EINES BESTANDTEILS AUS EINEM FLUID**
EXTRACTION COLUMN AND METHOD FOR EXTRACTING A COMPONENT FROM A FLUID
COLONNE D'EXTRACTION ET PROCÉDÉ D'EXTRACTION D'UN COMPOSANT D'UN FLUIDE

(30) Priorität: 18.12.2013 DE 102013226428; 18.12.2013 US 201361917368 P
(43) Veröffentlichungstag der Anmeldung: 26.10.2016
(73) Patentinhaber: BASF SE, 67056 Ludwigshafen am Rhein (DE)
(72) Erfinder: HAMMON, Ulrich, 68163 Mannheim (DE); PFEIFFER, Daniel, 67435 Neustadt (DE); WALTER, Jonathan, 69214 Eppelheim (DE)
(86) Internationale Anmeldenummer: PCT/EP2014/078193
(87) Internationale Veröffentlichungsnummer: WO 2015/091628

(56) Entgegenhaltungen:
- EP-A1- 1 088 577
- DE-A1- 10 002 806
- DE-A1- 10 115 277
- DE-C1- 10 135 585

## Beschreibung

Die vorliegende Erfindung betrifft eine Extraktionskolonne mit einem zumindest abschnittsweise zylindrischen, vertikal ausgerichteten Kolonnenkörper, der einen Kolonnenhohlraum bildet, der eine horizontale Maximalerstreckung aufweist. In dem Kolonnenkörper ist mindestens ein erster Zulauf für ein Extraktionsmittel, mindestens ein zweiter Zulauf für das zu extrahierende Fluid und mindestens jeweils ein Ablauf für das beladene Extraktionsmittel und das Raffinat ausgebildet. Des Weiteren betrifft die Erfindung ein Verfahren zum Extrahieren eines Bestandteils aus einem Fluid mittels einer Extraktionskolonne, die einen zylindrischen, vertikal ausgerichteten Kolonnenkörper aufweist, der einen Kolonnenhohlraum bildet, der eine horizontale Maximalerstreckung aufweist.

Bei der Extraktion wird eine Komponente, die auch als Übergangskomponente oder Extraktstoff bezeichnet wird, aus einem Stoffgemisch, das auch als Extraktionsgut oder als der zu extrahierende Stoff bezeichnet wird, mit Hilfe eines Extraktionsmittels abgetrennt. Bei der Extraktion werden die Komponenten des Extraktionsgutes nicht vollständig getrennt. Vielmehr geht der Extraktstoff in das Extraktionsmittel über und fällt als Extraktgemisch an. Als Raffinat bleibt das Extraktionsgut übrig, welches weniger Extraktstoff enthält.

Die vorliegende Erfindung betrifft insbesondere die Solventextraktion, bei der ein gelöster Extraktstoff aus flüssigen Extraktionsgütern durch selektiv wirkende flüssige Extraktionsmittel abgetrennt wird.

Eine solche Extraktion wird beispielsweise bei der Herstellung von Acrylsäure eingesetzt. Acrylsäure ist ein bedeutendes Zwischenprodukt, das beispielsweise im Rahmen der Herstellung von Polymerisatdispersionen (gegebenenfalls auch in Form ihrer Ester mit Alkanolen) sowie von Wasser superabsorbierenden Polymerisaten Verwendung findet.

Acrylsäure ist unter anderem durch heterogen katalysierte Gasphasen-Partialoxidation von C₃-Vorläufern (von C₃-Vorläuferverbindungen) der Acrylsäure (unter diesem Begriff sollen insbesondere solche chemischen Verbindungen zusammengefasst werden, die formal durch Reduktion von Acrylsäure erhältlich sind; bekannte C₃-Vorläufer von Acrylsäure sind z. B. Propan, Propen, Acrolein, Propionaldehyd und Propionsäure; der Begriff soll aber auch Vorläuferverbindungen der vorgenannten Verbindungen umfassen, wie z. B. Glycerin (von Glycerin ausgehend kann Acrylsäure beispielsweise durch heterogen katalysierte oxidative Dehydratisierung in der Gasphase erzeugt werden; vgl. z. B. EP-A 1 710 227, WO 06/114506 und WO 06/092272) mit molekularem Sauerstoff an im festen Aggregatzustand befindlichen Katalysatoren bei erhöhter Temperatur erhältlich.

Dabei werden die genannten Ausgangsgase, in der Regel mit inerten Gasen wie z. B. Stickstoff, CO₂, gesättigten Kohlenwasserstoffen und/oder Wasserdampf verdünnt, im Gemisch mit molekularem Sauerstoff bei erhöhten Temperaturen sowie gegebenenfalls erhöhtem Druck über (z. B. übergangsmetallische) Mischoxidkatalysatoren geleitet und oxidativ in ein Acrylsäure, Wasser sowie unerwünschten Nebenprodukte wie z. B. Furfurale, Benzaldehyd, Aceton, Formaldehyd und Maleinsäureanhydrid etc. enthaltendes Produktgasgemisch umgewandelt, aus welchem die Acrylsäure abgetrennt werden muss (die Nebenprodukte und die von Wasserdampf verschiedenen inerten Verdünnungsgase sollen in dieser Schrift unter dem Begriff "Nebenkomponenten" zusammengefasst werden; außerdem soll dieser Begriff die bei den Acrylsäureabtrennverfahren üblicherweise zugesetzten Polymerisationsinhibitoren umfassen).

Von Propionaldehyd und/oder Propionsäure ausgehend handelt es sich bei der heterogen katalysierten Gasphasen-Partialoxidation mit molekularem Sauerstoff wenigstens teilweise um eine oxidative Dehydrierung.

Aus den Schriften DE-A 199 24 533, DE-A 199 24 532, WO 01/77056, DE-A 101 56 016, DE-A 102 43 625, DE-A 102 23 058, DE-A 102 35 847, WO 2004/035514, WO 00/53560 und DE-A 103 32 758 sind wie eingangs beschriebene Verfahren zur Herstellung von Acrylsäure bekannt, bei denen eine Grundabtrennung einer rohen Acrylsäure durch fraktionierende Kondensation des Produktgasgemisches der heterogen katalysierten Gasphasen-Partialoxidation vorgenommen wird. Der Begriff rohe Acrylsäure bzw. Rohacrylsäure bringt dabei zum Ausdruck, dass es sich bei der über den ersten Seitenabzug entnommenen Acrylsäure um kein reines Produkt, sondern um ein Gemisch handelt, das neben Acrylsäure (in der Regel ≥ 50 oder ≥ 60 Gew.-%, meist ≥ 70 oder ≥ 80 Gew.-%, vielfach ≥ 90 Gew.-% und häufig ≥ 95 Gew.-% oder mehr des Gesamtgewichtes) noch Wasser und Nebenkomponenten wie z. B. niedere Aldehyde (z. B. Furfurale, Acrolein, Benzaldehyd), niedere Carbonsäuren (z. B. Essigsäure, Propionsäure, Ameisensäure) etc. enthält. In jedem Fall ist der Gesamtgehalt an Wasser und Nebenkomponenten, bezogen auf den Gehalt an Acrylsäure, in der rohen Acrylsäure geringer als im Produktgasgemisch der Gasphasen-Partialoxidation, weshalb man auch sagt, dass die rohe Acrylsäure diese Bestandteile insgesamt abgereichert enthält (einzelne Bestandteile können hingegen in der rohen Acrylsäure vergleichsweise angereichert enthalten sein).

Teilweise ist die Reinheit der so abgetrennten rohen Acrylsäure bereits für den ins Auge gefassten Verwendungszweck der Acrylsäure ausreichend (z. B. zum Zweck der Veresterung derselben, oder zum Zweck des Aufbaus von durch radikalischer Polymerisation erhältlichen Polymerisaten). Vielfach wird man die abgetrennte rohe Acrylsäure jedoch wenigstens einem weiteren thermischen Trennverfahren unterwerfen, um aus der rohen Acrylsäure eine reinere (eine einen im Vergleich zur rohen Acrylsäure höheren Acrylsäuregehalt in Gew.-% aufweisende) Acrylsäure zu gewinnen, die den für den jeweiligen Verwendungszweck erforderlichen Reinheitsgrad aufweist.

Unter thermischen Trennverfahren werden dabei solche verstanden, bei denen unter Zufuhr oder unter Entzug von (in der Regel thermischer) Energie ein physikalisch wenigstens zweiphasiges System erzeugt wird, wobei es infolge der zwischen den Phasen bestehenden Temperatur- und Stoffmengengradienten zu einem Wärme- und Stoffaustausch kommt, der letztlich die gewünschte Auftrennung bedingt, und die Extraktion.

Häufig werden thermische Trennverfahren in trennwirksame Einbauten enthaltenden Trennkolonnen durchgeführt, in denen die vorgenannten wenigstens zwei stofflichen Phasen in der Regel zueinander im Gegenstrom geführt werden. Vielfach ist eine der beiden stofflichen Phasen gasförmig (sie wird in einer Trennkolonne in der Regel als aufsteigende Phase geführt) und die andere flüssig (sie wird in einer Trennkolonne in der Regel als absteigende Phase geführt). Grundsätzlich können die wenigstens zwei stofflichen Phasen aber auch flüssig (z. B. im Fall einer Extraktion) oder fest und flüssig (z. B. im Fall einer Kristallisation), oder fest und gasförmig (z. B. im Fall einer Adsorption) sein.

Beispiele für Fallgestaltungen thermischer Trennverfahren, bei denen eine der wenigstens zwei stofflichen Phasen flüssig und eine gasförmig ist, und damit natürliches Element des in dieser Schrift verwendeten Begriffs "thermische Trennverfahren", sind die Rektifikation (eine aufsteigende Dampfphase wird in der Trennkolonne im Gegenstrom zu einer absteigenden Flüssigphase geführt), und die Desorption (der Umkehrprozess zur Absorption; das in einer Flüssigphase gelöste Gas wird durch Erniedrigung des Drucks über der Flüssigphase, durch Erhöhung der Temperatur der Flüssigphase und/oder durch Hindurchführen einer Gasphase durch die Flüssigphase aus der Flüssigphase herausgeführt; ist die Hindurchführung einer Gasphase beteiligt, wird die Desorption auch als Strippung bezeichnet). Aber auch die Absorption (in der Regel wird ein in einer Trennkolonne aufsteigendes Gas zu wenigstens einem in der Trennkolonne flüssig absteigenden Absorptionsmittel im Gegenstrom geführt) und die fraktionierende Kondensation eines Gasgemischs (Gas-/Flüssigphase Beispiel) sind Bestandteil des Begriffs thermisches Trennverfahren. Ein besonders günstiges thermisches Trennverfahren zur Weiterreinigung von roher Acrylsäure ist die kristallisative Weiterreinigung (die Kristallisation).

Nachteilig an den bekannten Verfahren zur Grundabtrennung einer rohen Acrylsäure durch fraktionierende Kondensation des Produktgasgemischs einer heterogen katalysierten Gasphasen-Partialoxidation wenigstens eines C₃-Vorläufers der Acrylsäure ist jedoch der zusätzliche Anfall von noch Acrylsäure und Nebenkomponenten enthaltendem saurem Wasser (vereinfacht auch "Sauerwasser" genannt). Die Bezeichnung "Sauerwasser" bringt dabei zunächst zum Ausdruck, dass das Sauerwasser in der Regel ≥ 50 Gew.-%, häufig ≥ 60 Gew.-%, vielfach ≥ 70 Gew.-% und oft ≥ 80 Gew.-% an Wasser enthält (dabei handelt es sich in der Regel sowohl um Reaktionswasser, als auch um als inertes Verdünnungsgas im Rahmen der Gasphasen-Partialoxidation mitverwendetes Verdünnungswasser (Wasserdampf)).

Sie bringt aber auch zum Ausdruck, dass es neben Wasser noch Nebenkomponentensäuren wie z. B. Propionsäure, Essigsäure und Ameisensäure sowie Acrylsäure enthält und damit einen pH-Wert von < 7 aufweist (der Gesamtgehalt der von Acrylsäure verschiedenen Nebenkomponentencarbonsäuren liegt in der Regel, bezogen auf das Gewicht des Sauerwassers, bei Werten ≤ 10 Gew.-%, teilweise bei Werten ≤ 5 Gew.-%).

Normalerweise wird der Acrylsäuregehalt des Sauerwassers 4 oder 5 bis 15, häufig ca. 10 Gew.-% betragen. Nachteilig an den im zitierten Stand der Technik empfohlenen Verfahren zur Grundabtrennung von Acrylsäure aus dem Produktgasgemisch der heterogen katalysierten Gasphasen-Partialoxidation ist, dass sie das nicht in die Rektifikationskolonne rückgeführte noch Acrylsäure enthaltende Sauerwasser in seiner Gesamtheit der Verbrennung zuführen (vgl. insbesondere DE-A 102 43 625, WO 2004/035514 und DE-A 103 32 758). Dies ist insofern von Nachteil, als die Sauerwasserverbrennung z. B. die Ausbeute am gewünschten Produkt Acrylsäure mindert.

In der DE 10 2007 055 086 A1 bzw. der WO 2008/090190 A1 wird daher ein Verfahren zur Herstellung von Acrylsäure vorgeschlagen, bei dem eine erhöhte Ausbeute an Acrylsäure dadurch gewährleistet wird, dass Acrylsäure aus dem Sauerwasser extrahiert und rückgeführt wird. Das Dokument DE 101 15 277 betrifft ein Verfahren zur kontinuierlichen Gewinnung von Acrylsäure mittels einer Multischaft-Kolonne für die destillative Trennung von Mehrstoffgemischen.

Die DE 10 2007 055 086 A1 beschreibt demgemäß ein Verfahren zur Herstellung von Acrylsäure, bei dem man durch heterogen katalysierte Gasphasen-Partialoxidation wenigstens eines C₃-Vorläufers (einer C₃-Vorläuferverbindung) der Acrylsäure mit molekularem Sauerstoff an im festen Aggregatzustand befindlichen Katalysatoren bei erhöhter Temperatur ein Acrylsäure, Wasserdampf und Nebenkomponenten enthaltendes Produktgasgemisch erzeugt. Dabei wird die Temperatur des Acrylsäure, Wasserdampf und Nebenkomponenten enthaltenden Produktgasgemischs gegebenenfalls durch direkte (durch direkten Kontakt mit einer Kühlflüssigkeit) und/oder indirekte Kühlung verringert. Das Acrylsäure, Wasserdampf und Nebenkomponenten enthaltende Produktgasgemisch wird anschließend in eine mit trennwirksamen Elementen ausgerüstete Kondensationskolonne geleitet. Das Produktgasgemisch steigt anschließend innerhalb der Kondensationskolonne in sich selbst auf und wird dabei fraktionierend kondensiert. Über einen ersten, oberhalb der Zuführstelle des Produktgasgemischs in die Kondensationskolonne befindlichen Seitenabzug wird eine Wasser und Nebenkomponenten insgesamt entreichert enthaltende rohe Acrylsäure als Zielprodukt aus der Kondensationskolonne herausführt. Über einen zweiten, oberhalb das ersten Seitenabzugs befindlichen Flüssigphasenabzug (vorzugsweise einen Seitenabzug; alle Aussagen in dieser Schrift sind insbesondere gültig im Fall eines solchen Sauerwasserseitenabzugs) wird saures Wasser (Sauerwasser), das noch Acrylsäure und Nebenkomponenten enthält, aus der Kondensationskolonne herausführt. Am Kopf der Kondensationskolonne wird ein tiefer (bei niedrigerer Temperatur (bezogen auf Atmosphärendruck)) als Wasser siedende Nebenkomponenten enthaltendes Restgasgemisch sowie aus dem Sumpfraum der Kondensationskolonne eine noch Acrylsäure und schwerer als Acrylsäure siedende Folgeprodukte und Nebenkomponenten enthaltende Sumpfflüssigkeit aus der Kondensationskolonne herausführt. Eine Teilmenge des entnommenen sauren Wassers wird als solches und/oder nach Abkühlung desselben als Rücklaufflüssigkeit in die Kondensationskolonne rückführt. Die rohe Acrylsäure wird gegebenenfalls wenigstens einem weiteren thermischen Trennverfahren zum Zweck ihrer Weiterreinigung unterworfen. Gemäß dem Verfahren nimmt man wenigstens bei einer Teilmenge von nicht in die Kondensationskolonne rückgeführtem saurem Wasser in diesem enthaltene Acrylsäure durch Extraktion mit einem organischen Lösungsmittel unter Ausbildung eines Acrylsäure enthaltenden organischen Extraktes aus dem sauren Wasser heraus in das organische Lösungsmittel auf. Nachfolgend wird die Acrylsäure aus dem organischen Extrakt unter Anwendung wenigstens eines thermischen Trennverfahrens abgetrennt und vom Extrakt abgetrennte Acrylsäure wird in die Kondensationskolonne zurückführt, oder der Weiterreinigung der rohen Acrylsäure zuführt und/oder in die wässrige Lösung eines Metallhydroxids aufgenommen.

Der Innendurchmesser der in der DE 10 2007 055 086 A1 angegebenen Extraktionskolonne beträgt 800 mm. Um größere Mengen Sauerwasser verarbeiten zu können, besteht das Bedürfnis, auch größere Extraktionskolonnen zu verwenden, die insbesondere einen größeren Innendurchmesser aufweisen. Dabei hat es sich herausgestellt, dass sich die Ausbeute der Rückgewinnung der Acrylsäure aus dem Sauerwasser verschlechtert, wenn der Innendurchmesser der Extraktionskolonne zu groß wird. Es wurde daher vorgeschlagen, größere Mengen von Sauerwasser mittels parallel geschalteter dünnerer Extraktionskolonnen zu verarbeiten. Dies ist jedoch aus Kostengründen nachteilig.

Der vorliegenden Erfindung lag daher die Aufgabe zugrunde, eine hinsichtlich axialer Rückvermischung verbesserte Extraktionskolonne und ein verbessertes Verfahren zum Extrahieren eines Bestandteils aus einem Fluid mittels einer Extraktionskolonne zur Verfügung zu stellen, die insbesondere dadurch ausgezeichnet sind, dass sie eine erhöhte Ausbeute des Bestandteils bei der Extraktion auch dann gewährleistet, wenn die Extraktionskolonne einen großen Durchmesser aufweist.

Erfindungsgemäß wird diese Aufgabe durch eine Extraktionskolonne mit den Merkmalen des Anspruchs 1 und ein Verfahren mit den Merkmalen des Anspruchs 14 gelöst. Vorteilhafte Ausgestaltungen und Weiterbildungen ergeben sich aus den abhängigen Ansprüchen.

Demgemäß wurde eine Extraktionskolonne gefunden, die einen zumindest abschnittsweise zylindrischen, vertikal ausgerichteten Kolonnenkörper aufweist, der einen Kolonnenhohlraum bildet, der eine horizontale Maximalerstreckung aufweist. In dem Kolonnenkörper ist mindestens ein erster Zulauf für ein Extraktionsmittel, mindestens ein zweiter Zulauf für das zu extrahierende Fluid und mindestens ein Ablauf für das Extraktgemisch und mindestens einen Ablauf für das Raffinat ausgebildet. Die erfindungsgemäße Extraktionskolonne ist dadurch gekennzeichnet, dass in dem Kolonnenhohlraum eine vertikal ausgerichtete Trenneinrichtung angeordnet ist, die den Kolonnenhohlraum in mehrere vertikal ausgerichtete und horizontal getrennte Bereiche unterteilt. Dabei ist die horizontale Maximalerstreckung jedes Bereichs kleiner als die horizontale Maximalerstreckung des Kolonnenhohlraums.

Unter der horizontalen Maximalerstreckung wird in dieser Schrift der größte Abstand zwischen zwei beliebigen Punkten der inneren Oberfläche des Kolonnenkörpers bzw. der Oberfläche der Trennwand in einer bestimmten horizontalen Ebene durch den Kolonnenkörper verstanden. Klarstellend wird angemerkt, dass die Verbindungsstrecke zwischen den zwei beliebigen Punkten nicht vollständig in dem Bereich liegen muss, für den die horizontale Maximalerstreckung ermittelt wird. Sie kann auch einen anderen Bereich kreuzen. Somit ist bei einem kreiszylindrischen Kolonnenkörper die horizontale Maximalerstreckung des Kolonnenkörpers der Innendurchmesser des Kolonnenkörpers. Wenn der Kolonnenhohlraum durch die Trenneinrichtung unterteilt ist, ist die horizontale Maximal-erstreckung eines durch die Trenneinrichtung gebildeten Bereichs der größte Abstand zwischen zwei beliebigen Punkten der inneren Oberfläche des Kolonnenkörpers und/oder der Oberfläche der Trennwand, die jeweils den Bereich begrenzen, in einer bestimmten horizontalen Ebene.

Unter einer Trenneinrichtung wird in dieser Schrift eine Einrichtung verstanden, welche eine Querströmung, d. h. eine Strömung in einer Horizontalebene von einem Bereich in den anderen Bereich, verhindert. Es findet somit in der Extraktionskolonne kein horizontaler Stofftransport von einem Bereich in den anderen Bereich statt. Eine Querströmung zum Stofftransport kann allenfalls innerhalb eines Bereichs erfolgen. Ferner findet selbstverständlich ein Stofftransport in vertikaler Richtung statt.

Es wird davon ausgegangen, dass die Verschlechterung der Extraktausbeute bei der Extraktion mit Extraktionskolonnen, die einen größeren Durchmesser haben, durch eine horizontale Querströmung des zu extrahierenden Fluids verursacht wird. Es wird angenommen, dass diese Querströmung durch Axialwirbel verursacht wird, die sich bei einem größeren Innendurchmesser der Extraktionskolonne bilden. Dieses Problem wird erfindungsgemäß durch die vertikale Unterteilung des Kolonnenhohlraums mittels der Trenneinrichtung gelöst. Um nachteilige Axialwirbel zu vermeiden, ist es dabei wichtig, dass die Trenneinrichtung den Kolonnenhohlraum nicht nur vertikal in getrennte Bereiche unterteilt, sondern dass auch die horizontale Maximalerstreckung im Kolonnenhohlraum durch die Trenneinrichtung verringert wird.

Gemäß einer Ausgestaltung der erfindungsgemäßen Extraktionskolonne ist die horizontale Maximalerstreckung jedes Bereichs bei jedem Horizontalschnitt durch den Kolonnenkörper, welcher die Trenneinrichtung schneidet, kleiner als die horizontale Maximalerstreckung des Kolonnenhohlraums. Die Trenneinrichtung erstreckt sich insbesondere in vertikaler Richtung über einen Mittelabschnitt des Kolonnenhohlraums. Oberhalb der Trenneinrichtung ist der Kolonnenkopf gebildet, unterhalb ist der Kolonnensumpf gebildet. Die Trenneinrichtung erstreckt sich gegebenenfalls nicht in den Kolonnenkopf und in den Kolonnensumpf hinein. Betrachtet man nun die Geometrie im Bereich der Trenneinrichtung, wird durch die Trenneinrichtung die horizontale Maximalerstreckung im Kolonnenhohlraum beim jedem Horizontalschnitt verkleinert.

Die Ausbildung der Trenneinrichtung hängt im Wesentlichen von der Geometrie des horizontalen Querschnitts des Kolonnenkörpers ab. Üblicherweise ist dieser Querschnitt kreisförmig. Die horizontale Maximalerstreckung des Kolonnenhohlraums ist in diesem Fall der Innendurchmesser des Kolonnenkörpers. In einem solchen Fall führt eine vertikale Halbierung des Kolonnenhohlraums durch eine Trenneinrichtung nicht zu einer Verringerung der horizontalen Maximalerstreckung in dem Kolonnenhohlraum. Bei der Seite einer solchen Trenneinrichtung wäre die horizontale Maximalerstreckung der beiden Bereiche gleich dem Innendurchmesser des Kolonnenkörpers. Die geringfügige Verringerung der Maximalerstreckung, welche sich durch die Wandstärke der Trenneinrichtung ergeben würde, wird in diesem Fall vernachlässigt.

Erfindungsgemäß ist das Verhältnis der größten horizontalen Maximalerstreckung eines Bereichs zu der horizontalen Maximalerstreckung des Kolonnenhohlraums kleiner als 0,95, insbesondere kleiner als 0,9 und bevorzugt kleiner als 0,75.

Wenn der Kolonnenkörper bei einem horizontalen Schnitt kreisförmig ist, unterteilt die Trenneinrichtung beispielsweise den Kolonnenhohlraum in zumindest drei vertikal aufgerichtete und horizontal getrennte Bereiche. Die Trenneinrichtung kann beispielsweise drei vertikale Trennbleche umfassen, die sich jeweils von der Mitte des Kolonnenhohlraums radial zum Kolonnenkörper erstrecken, wobei sie insbesondere einen Winkel von 120° einschließen.

Des Weiteren kann die Trenneinrichtung den Kolonnenhohlraum in vier vertikal ausgerichtete und horizontal getrennte Bereiche unterteilen. Beispielsweise können in diesem Fall zwei kreuzweise angeordnete Trennbleche, die einen Winkel von 90° einschließen, den Kolonnenhohlraum in vier gleich große Kreissegmente unterteilen.

Gemäß einer anderen Ausgestaltung wird von der Trenneinrichtung ein zentraler Mittelbereich gebildet, der beabstandet von dem Kolonnenkörper angeordnet ist. Zwischen dem Mittelbereich und dem Kolonnenkörper sind zumindest zwei weitere Bereiche gebildet. Bevorzugt sind zwischen dem Mittelbereich und dem Kolonnenkörper mehrere weitere Bereiche gebildet. Diese können sich z. B. radial von dem Kolonnenkörper nach innen bis zu der Trennwand erstrecken, die den zentralen Mittelbereich bildet. Der zentrale Mittelbereich kann in diesem Fall einen kreisförmigen Querschnitt aufweisen. Dieser kreisförmige Querschnitt ist insbesondere konzentrisch zu dem kreisförmigen Querschnitt des Kolonnenkörpers. Der Mittelbereich kann im Querschnitt jedoch auch die Form eines Vielecks, insbesondere eines gleichseitigen Vielecks, besitzen, wobei sich die radial ausgerichteten Trennbleche von den Ecken des Vielecks zum Kolonnenkörper erstrecken.

Bei den beschriebenen Ausgestaltungen der Trenneinrichtung wird jeweils die horizontale Maximalerstreckung des Kolonnenhohlraums in unterschiedlichem Maße verringert. Die durch die Trenneinrichtung gebildeten, voneinander getrennten Bereiche weisen eine wesentlich geringere horizontale Maximalerstreckung auf. Die nachteilige Querströmung in dem Kolonnenhohlraum wird somit wirksam von der Trenneinrichtung unterbunden.

Bevorzugt sind die von der Trenneinrichtung gebildeten Bereiche jeweils gleich groß. Hierdurch wird erreicht, dass sich in den Bereichen jeweils im Wesentlichen das gleiche Volumen des zu extrahierenden Fluids befindet.

Außerdem können die von der Trenneinrichtung gebildeten Bereiche bei jedem horizontalen Schnitt durch den Kolonnenkörper, der die Trenneinrichtung schneidet, die gleiche Geometrie haben. Die bei den Horizontalschnitten gebildeten Flächen der Bereiche sind insbesondere identisch. Dies hat den Vorteil, dass bei den Bereichen identische Packungen eingesetzt werden können, wie es später erläutert wird. Dies führt zu einer Kostensenkung bei der Herstellung der erfindungsgemäßen Extraktionskolonne.

Gemäß einer weiteren Ausgestaltung der erfindungsgemäßen Extraktionskolonne münden die Bereiche bei ihren oberen Enden in einen gemeinsamen Kolonnenkopf und bei ihren unteren Enden in einen gemeinsamen Kolonnensumpf. Im Bereich des Kolonnenkopfes und dem Bereich des Kolonnensumpfes ist die horizontale Erstreckung des Kolonnenhohlraums somit nicht durch eine Trenneinrichtung unterteilt. Die Bereiche sind somit parallel geschaltet. Beim Kolonnenkopf ist einer der Zuläufe und gegebenenfalls ein Ablauf angeordnet. Beim Kolonnensumpf ist der andere der Zuläufe und gegebenenfalls ein Ablauf angeordnet.

Die erfindungsgemäße Extraktionskolonne besitzt insbesondere einen großen Durchmesser. Der Innendurchmesser des Kolonnenkörpers, d. h. die horizontale Maximalerstreckung des Kolonnenhohlraums, ist beispielsweise größer oder gleich 800 mm, insbesondere größer oder gleich 1000 mm. Es sind jedoch auch Kolonnendurchmesser von über 2000 mm oder 5000 mm möglich.

Die Höhe des Kolonnenkörpers ist beispielsweise größer als 5 m, insbesondere größer als 10 m. Es ist jedoch auch möglich, dass die Höhe des Kolonnenkörpers 30 m oder 40 m übersteigt.

Anwendungstechnisch zweckmäßig sind in dem Kolonnenhohlraum trennwirksame Einbauten angeordnet. Durch die trennwirksamen Einbauten wird die Stofftrennung in der Extraktionskolonne verbessert. Die Einbauten sind für die durch die Trenneinrichtung gebildeten Bereiche separat voneinander vorgesehen, d. h. die Trenneinrichtung trennt auch die Einbauten voneinander, so dass auch über die Einbauten kein Stoffaustausch zwischen den von der Trenneinrichtung gebildeten Bereichen möglich ist.

Die trennwirksamen Einbauten sind insbesondere so ausgebildet, dass sich der Stoffaustausch zwischen einer disperse Tropfenphase und einer kontinuierlichen Phase verbessert. Die kontinuierliche Phase ist dabei insbesondere keine benetzende Filmphase. Die trennwirksamen Einbauten sind insbesondere so ausgebildet, dass sie Flüssigkeitstropfen bilden. Dabei wird die von den Flüssigkeitstropfen bereitgestellte Oberfläche ständig durch Spaltung und Koaleszenz neu gebildet bzw. erneuert. Da bei der Koaleszenz die Emulsionströpfchen zusammentreffen und verschmelzen, vergrößern sich die Tröpfchen, wohingegen sich die von der dispersen Phase gebildete Oberfläche verkleinert. Die trennwirksamen Einbauten spalten die verschmolzenen Tröpfchen wieder in kleinere Tröpfchen auf, so dass sich die von der dispersen Phase gebildete Oberfläche wieder vergrößert.

Die Einbauten können beispielsweise in Form von Packungen, insbesondere strukturierten bzw. geordneten Packungen, Füllkörper und/oder Schüttungen vorgesehen sein. Unter den Schüttungen sind solche mit Ringen, Wendeln, Sattelkörpern, Raschig-, Intos- oder Pall-Ringen, Barrel- oder Intalox-Sätteln, Top-Pak etc. bevorzugt. Für erfindungsgemäß zu verwendende Extraktionskolonnen besonders geeignete Packungen sind z. B. Packungen der Julius Montz GmbH in D-40705 Hilden, wie z. B. die Packung Montz-Pak B1-350. Vorzugsweise verwendet man gelochte strukturierte Packungen aus Edelstahlblechen. Packungskolonnen mit geordneten Packungen sind dem Fachmann an sich bekannt und z. B. in Chem.-Ing.Tech. 58 (1986) Nr. 1, S. 19 - 31 sowie in der Technischen Rundschau Sulzer 2/1979, S. 49 ff. der Gebrüder Sulzer Aktiengesellschaft in CH-Winterthur beschrieben.

Des Weiteren sind auch Extraktionskolonnen mit Einbauten in Form von Böden geeignet, wobei hier zwischen pulsierten Siebbodenkolonnen und Querstromsiebbodenkolonnen unterschieden werden muss. Bei den pulsierten Siebbodenkolonnen werden beide Phasen durch die Durchtrittsöffnungen (in der Regel Löcher, d. h., kreisrunde Durchtrittsöffnungen) im Siebboden geführt. Beim Aufwärtshub der Pulsation wird die leichtere Phase nach oben durch die Löcher des Siebbodens gedrückt, beim Abwärtshub entsprechend die schwere Phase nach unten. Derartige Kolonnen sind in Pilhofer/Mewes "Siebboden-Extraktionskolonnen: Vorausberechnung unpulsierter Kolonnen" Verlag Chemie Weinheim, New York 1979 ISBN 3-527-25837-X beschrieben. Bei Verwendung von Querstromsiebbodenkolonnen läuft die kontinuierliche Phase über Ablaufschächte von einem Boden zum nächsten Boden und nur die disperse Phase wird auf Grund der Dichtedifferenz durch die Löcher der Siebböden gedrückt. Ein weiterer Kolonnentyp sind Extraktionskolonnen mit bewegten Rührern wie sie unter der Bezeichnung Kühni-Kolonne von der Firma Sulzer angeboten werden.

Gemäß einer Weiterbildung der erfindungsgemäßen Extraktionskolonne sind die trennwirksamen Einbauten in den von der Trenneinrichtung gebildeten Bereichen aus identischen Einzelpackungen gebildet. Die Geometrie eines horizontalen Schnitts durch eine Einzelpackung entspricht dabei der Geometrie eines horizontalen Schnitts durch den Bereich, in den die Einzelpackung anzuordnen ist. Eine Einzelpackung lässt sich daher in vertikaler Richtung in den Bereich einführen. Hierdurch werden die Herstellungs- und Montagekosten der Extraktionskolonne verringert.

Gemäß einer Weiterbildung der erfindungsgemäßen Extraktionskolonne ist die Trenneinrichtung in Vertikalsegmente unterteilt, die in dem Kolonnenhohlraum aufeinander gesetzt sind. Die Höhe der Einzelpackung kann dabei der Höhe eines Vertikalsegments der Trenneinrichtung entsprechen. Ferner ist es möglich, dass die Höhe eines Vertikalsegments etwas größer oder etwas kleiner als die Höhe eines Vertikalsegments der Trenneinrichtung ist. Durch diese Maßnahmen wird die Montage der Extraktionskolonne vereinfacht.

Gemäß einer bevorzugten Weiterbildung der erfindungsgemäßen Extraktionskolonne ist auf der Innenseite des Kolonnenkörpers zumindest eine vertikal ausgerichtete Nut gebildet, in welche die Trenneinrichtung eingreift. Auf diese Weise wird die Trenneinrichtung verdrehsicher im Kolonnenhohlraum gelagert. Dies ist insbesondere dann von Vorteil, wenn der Kolonnenkörper einen kreisförmigen horizontalen Querschnitt aufweist.

Die Ausbildung einer Nut auf der Innenseite des Kolonnenkörpers ist gegenüber der Ausbildung eines Vorsprungs bei der Innenseite des Kolonnenkörpers bevorzugt, da ein Vorsprung in einen von der Trenneinrichtung gebildeten Bereich hineinragen würde, so dass dieser Vorsprung bei den Einbauten ausgespart werden müsste. Dies wäre bei der Herstellung der Einbauten kostenintensiv. Bei der erfindungsgemäßen Ausbildung der Innenseite des Kolonnenkörpers mit einer Nut, ist es nicht erforderlich, eine Aussparung bei den Einbauten vorzunehmen. Die Einbauten lassen sich daher kostengünstiger herstellen.

Gemäß einer weiteren Ausgestaltung sind auf der Innenseite des Kolonnenkörpers mehrere Nuten ausgebildet, in die Trennbleche der Trenneinrichtung eingreifen. Beispielsweise können alle Trennbleche, welche bis zur Innenseite des Kolonnenkörpers reichen, in Nuten eingreifen, die auf der Innenseite des Kolonnenkörpers gebildet sind.

Für den Eingriff der Trenneinrichtung in eine Nut oder mehrere Nuten in der Innenwand des Mantels, kann die Trenneinrichtung zumindest drei Schichten umfassen. Dabei steht die mittlere Schicht gegenüber den äußeren Schichten vor. Diese mittlere Schicht greift dann in eine Nut ein. Die äußeren Schichten sind mit ihren Stirnseiten dann in direkter Nachbarschaft zu der Innenseite des Kolonnenkörpers jeweils neben der Nut. Sie stoßen insbesondere fluiddicht an diese Innenseite an. Durch diesen symmetrischen Aufbau der Trenneinrichtung wird gewährleistet, dass die von der Trenneinrichtung gebildeten Bereiche identische Querschnittsflächen besitzen.

Bei dem erfindungsgemäßen Verfahren zum Extrahieren eines Bestandteils aus einem Fluid mittels einer Extraktionskolonne, die einen zylindrischen, vertikal ausgerichteten Kolonnenkörper aufweist, der einen Kolonnenhohlraum bildet, der eine horizontale Maximalerstreckung aufweist, wird das zu extrahierenden Fluid in mehrere vertikal ausgerichtete und horizontal getrennte Bereiche eingeleitet, die in den Kolonnenhohlraum von einer vertikal ausgerichteten Trenneinrichtung gebildet werden, die den Kolonnenhohlraum in die Bereiche unterteilt, wobei die horizontale Maximalerstreckung jedes Bereichs kleiner als die horizontale Maximalerstreckung des Kolonnenhohlraums ist. Bei dem Verfahren wird ferner ein Extraktionsmittel in die Bereiche des Kolonnenhohlraums eingeleitet. Der Bestandteil des zu extrahierenden Fluids wird dann in den Bereichen des Kolonnenhohlraums von dem Extraktionsmittel aufgenommen und als Extraktgemisch entnommen.

Bei dem erfindungsgemäßen Verfahren wird die horizontale Querströmung sowohl des zu extrahierenden Fluids, d. h. des Fluidgemisches, als auch des Extraktionsmittels durch die Trenneinrichtung unterbunden. Hierdurch wird die Ausbeute des Extrakts insbesondere dann gesteigert, wenn der Innendurchmesser der Extraktionskolonne relativ groß ist. Wie vorstehend beschrieben, wird angenommen, dass auch bei dem erfindungsgemäßen Verfahren durch die Trenneinrichtung verhindert wird, dass Axialwirbel entstehen, die zu einer Querströmung des Fluidgemisches führen.

Erfindungsgemäß vorteilhaft werden wenigstens 25%, besser wenigstens 50%, noch besser wenigstens 75% und bevorzugt die Gesamtmenge des den zu extrahierenden Bestandteil enthaltenen Stoffstroms erfindungsgemäß extrahiert. Dabei werden durch die erfindungsgemäße Ausführung der Extraktionskolonne Abreicherungsgrade von bis zu 99,99% bezüglich des zu extrahierenden Stoffes erreicht.

Bei dem erfindungsgemäßen Verfahren tritt anwendungstechnisch zweckmäßig die spezifisch schwerere Phase oben und die spezifisch leichtere Phase unten in die Extraktionskolonne ein. In der Kolonne bewegen sich beide Phasen im Gegenstrom.

Beispielsweise wird bei dem erfindungsgemäßen Verfahren das zu extrahierende Fluid zunächst von unten in den Kolonnenhohlraum der Extraktionskolonne gefüllt. Von dort gelangt es in die von der Trenneinrichtung getrennten Bereiche im Kolonnenhohlraum. Das Extraktionsmittel wird oben in den Kolonnenkopf eingeleitet. Dort wird es tropfenförmig den Bereichen des Kolonnenhohlraums zugegeben. Das Extraktgemisch wird dann unten im Kolonnensumpf entnommen.

In dem Kolonnenhohlraum der Extraktionskolonne sind insbesondere trennwirksame Einbauten angeordnet. Diese bewirken einen verbesserten Stoffaustausch zwischen einer disperse Tropfenphase und einer kontinuierlichen Phase. Bei den trennwirksamen Einbauten bilden sich Flüssigkeitstropfen, so dass die von den Flüssigkeitstropfen bereitgestellte Oberfläche ständig durch Spaltung und Koaleszenz neu gebildet bzw. erneuert wird. Bei den trennwirksamen Einbauten spalten sich verschmolzene Tröpfchen wieder in kleinere Tröpfchen auf, so dass sich die von der dispersen Phase gebildete Oberfläche wieder vergrößert. Mittels der trennwirksamen Einbauten werden somit Flüssigkeitstropfen des Extraktionsmittels oder des zu extrahierenden Fluids gebildet.

Bei dem erfindungsgemäßen Verfahren ist insbesondere das Verhältnis des Extraktionsmittels zu dem zu extrahierenden Fluid bei den Bereichen über den Querschnitt der Extraktionskolonne im Wesentlichen gleich. Das Verhältnis des Extraktionsmittels zu dem zu extrahierenden Fluid weicht über den Querschnitt insbesondere um weniger als 30%, bevorzugt um weniger 10 %, besonders bevorzugt um weniger als 5 %, voneinander ab. Durch diese Ausgestaltung des Verfahrens ergibt sich eine geringere axiale Rückvermischung in der Kolonne und damit auch eine größere realisierbare Trennstufenzahlen. Auf diese Weise können somit die Extraktionsausbeuten gesteigert werden.

Des Weiteren sind bei dem erfindungsgemäßen Verfahren die Zusammensetzung des Extraktionsmittels und/oder die Zusammensetzung des zu extrahierenden Fluids über den Querschnitt der Extraktionskolonne im Wesentlichen gleich, d. h. es bleibt die Verteilung der beiden Phasen über den Querschnitt im Wesentlichen konstant. Die Abweichung der Dispersität ist beispielsweise weniger als 10%, insbesondere weniger als 5%. Auch hierdurch kann die Extraktionsausbeute gesteigert werden.

Das Extraktionsmittel, welches bei dem erfindungsgemäßen Verfahren verwendet wird, ist insbesondere ein organisches Lösungsmittel. Bei dem zu extrahierenden Fluid handelt es sich insbesondere um Acrylsäure und Essigsäure enthaltendes Wasser, d. h. Sauerwasser bzw. saures Wasser.

Erfindungsgemäß vorteilhaft wird das Extraktionsmittel einen höheren Siedepunkt als Acrylsäure (jeweils bezogen auf Atmosphärendruck) haben, da dies in der Regel die nachfolgende Abtrennung der Acrylsäure aus einem organischen Extrakt erleichtert.

Im Rahmen der erfindungsgemäß durchzuführenden Extraktion besitzt das als Extraktionsmittel zu verwendende organische Lösungsmittel insbesondere eine nennenswert höhere Viskosität als Wasser. In diesem Fall ist es erfindungsgemäß vorteilhaft, wenn das in die Extraktionskolonne eintretende organische Extraktionsmittel als disperse Phase vorliegt und das Sauerwasser als kontinuierliche Phase (dies bedingt z. B. einen beschleunigten Stoffaustausch zwischen den beiden Phasen und ermöglicht bei gleichem Trennergebnis letztlich kürzere Kolonnen; auch benetzt eine kontinuierliche wässrige Phase aus Edelstahl gefertigte Extraktionskolonnen und deren Einbauten besser; darüber hinaus führt ein Transport des zu extrahierenden Stoffes aus der kontinuierlichen Phase in die disperse Phase zu einer Stabilisierung der letzteren (geringere Koaleszenzneigung)). Bei Verwendung eines organischen Extraktionsmittel mit einer höheren Massendichte als der Massendichte des Sauerwassers bedeutet dies, dass das Extraktionsmittel am Kopf der Kolonne, insbesondere in Form von Tropfen, aufgegeben und dispergiert wird und die dabei resultierenden Extraktionsmitteltropfen in der Kolonne nach unten sinken. Im umgekehrten Fall, d.h., bei der Verwendung eines Extraktionsmittels mit einer geringeren Massendichte als der Massendichte von Sauerwasser, wird das Extraktionsmittel im Sumpf der Kolonne dispergiert und die dabei resultierenden Extraktionsmitteltropfen steigen in der Kolonne auf. Bei den bisher genannten Typen von Extraktionskolonnen mit Einbauten in Form von Packungen und/oder Füllkörpern sollte die unzerteilte kontinuierliche Phase die ausgewählten Einbauten gut benetzen, da andernfalls die Tropfen der dispersen Phase in der Regel an den Einbauten entlang kriechen.

In einfachster Weise wird man das organische Extraktionsmittel dabei mittels über den Kolonnenquerschnitt angeordnete und sich über die jeweilige Querschnittslänge der normalerweise kreiszylindrischen Extraktionskolonne erstreckende, in der Regel kreisförmige Durchtrittsöffnungen (Bohrungen) aufweisende Rohre (die normalerweise einen identischen Querschnitt aufweisen; man spricht auch von Rohrverteilern) aufgeben. Wird das organische Extraktionsmittel am Kopf der Kolonne aufgegeben weisen die kreisförmigen Durchtrittsöffnungen nach unten, bei Aufgabe des Extraktionsmittels am Sumpf der Kolonne nach oben. Der Durchmesser (die Längsausdehnung) der vorgenannten Durchtrittsöffnungen wird dabei üblicherweise 1 mm bis 10 mm, vorzugsweise 3 mm bis 6 mm und vielfach 2 bis 5 mm betragen. Dabei lässt man das Extraktionsmittel in einfacher Weise in die Verteilerrohre ein- und aus den Durchtrittsöffnungen wieder ausströmen.

Triebkraft für die Trennung von Extrakt und Raffinat ist der Unterschied in der Massendichte (g/cm³) zwischen beiden Phasen. Eine hohe Massendichtedifferenz der beiden flüssigen Phasen erleichtert die Phasentrennung und mindert Emulsionsbildung.

Mit Vorteil werden für die erfindungsgemäß durchzuführende Extraktion daher organische Lösungsmittel verwendet, deren Massedichte in kg/m³ sich von der Massendichte von Wasser (ebenfalls in kg/m³) um ≥ 25 kg/m³, vorzugsweise um ≥ 50 kg/m³ unterscheidet (bezogen auf den bei der Extraktion angewandten Druck und die bei der Extraktion angewandte Temperatur). In der Regel wird der vorgenannte Massendichteunterschied jedoch ≤ 250 kg/m³, in der Regel ≤ 150kg/m³ betragen.

Des Weiteren ist es für das erfindungsgemäße Verfahren günstig, wenn die dynamische Viskosität des organischen Extraktionsmittels unter den Extraktionsbedingungen ≤ 100 mPa•s, vorzugsweise ≤ 50 mPa•s beträgt. In der Regel wird die vorgenannte dynamische Viskosität jedoch ≥ 1 mPa•s betragen. Erfindungsgemäß besonders günstig sind dynamische Viskositäten im Bereich von 2 bis 10 mPa•s.

Weiterhin ist es beim erfindungsgemäßen Verfahren vorteilhaft, wenn die Grenzflächenspannung zwischen den beiden fluiden Phasen vergleichsweise hoch ist. Vor dem Hintergrund des bisher gesagten kommen als erfindungsgemäß geeignete Extraktionsmittel für die Sauerwasserextraktion unter anderem organische Flüssigkeiten in Betracht, deren Siedepunkt bei Normaldruck (1 atm) oberhalb von 150 bzw. oberhalb von 160 °C liegt. Beispielhaft genannt seien Mittelölfraktionen aus der Paraffindestillation, Diphenylether, Diphenyl, oder Mischungen der vorgenannten Flüssigkeiten, wie z. B. ein Gemisch aus 70 bis 75 Gew.-% Diphenylether und 25 bis 30 Gew.-% Diphenyl. Günstig ist auch die Verwendung eines Gemisches bestehend aus einer Mischung aus 70 bis 75 Gew.-% Diphenylether und 25 bis 30 Gew.-% Diphenyl, sowie bezogen auf des Gemisch, 0,1 bis 25 Gew.-% o-Dimethylphthalat.

Erfindungsgemäß besonders bevorzugte organische Lösungsmittel für die Sauerwasserextraktion sind die Ester von aliphatischen oder aromatischen Mono- oder Dicarbonsäuren (insbesondere wenn beide Carboxylgruppen verestert sind), deren alkoholische Komponente 1 bis 8 C-Atome und deren Carbonsäurekomponente 5 bis 20 C-Atome enthält. Vorzugsweise weist die alkoholische Komponente vor der Veresterung lediglich zwei oder nur eine Hydroxylgruppe auf. Besonders bevorzugt handelt es sich bei der alkoholischen Komponente um einwertige (eine OH-Gruppe) oder um zweiwertige (zwei OH-Gruppen) Alkanole. Mit Vorteil beträgt die Anzahl der C-Atome der alkoholischen Komponente (insbesondere im Fall von einwertigen oder zweiwertigen Alkanolen) 1 bis 6, besonders bevorzugt 1 bis 4 und ganz besonders bevorzugt 1 oder 2. Die aliphatischen oder aromatischen Mono- oder Dicarbonsäuren enthalten mit Vorteil 5 bis 15 C-Atome, bevorzugt 5 bis 10 C-Atome und besonders bevorzugt 6 bis 8 C-Atome (insbesondere im Fall einer jeweiligen Veresterung (auch im Diesterfall) mit 1 bis 4 bzw. mit 1 oder 2 C-Atome aufweisenden Alkanolen). Dicarbonsäuren sind gegenüber Monocarbonsäuren als Säurekomponente der relevanten Ester bevorzugt (insbesondere wenn beide Carboxylgruppen verestert sind). Phthalsäure, isoPhthalsäure und Terephthalsäure sowie Adipinsäure sind erfindungsgemäß ganz besonders bevorzugte Säurekomponenten der relevanten Ester. Das letztere trifft insbesondere im Fall der Dialkylester (C₁- bis C₈-Alkyl, vorteilhaft C₁-bis C₆-Alkyl, ganz besonders vorteilhaft C₁- bis C₄-Alkyl und noch besser C₁- oder C₂-Alkyl) zu. D. h., für das erfindungsgemäße Verfahren ganz besonders günstige Extraktionsmittel sind das Dimethylphthalat, das Diethylphthalat (z. B. Palatinol® A der BASF Aktiengesellschaft), das Dimethylisophthalat, das Diethylisophthalat, das Dimethylterephthalat, das Diethylterephthalat, der Adipinsäuredimethylester und der Adipinsäurediethylester.
Weitere für die erfindungsgemäße Sauerwasserextraktion geeignete Ester sind die Triester der Phosphorsäure, wie z. B. Tributylphosphat oder Trikresylphosphat. Als Kresylrest kommen dabei sowohl das ortho-Kresyl, das meta-Kresyl- und das para-Kresyl in Betracht. Außerdem kommen als Extraktionsmittel für die erfindungsgemäße Sauerwasserextraktion Ester aus Acrylsäure und verzweigten oder linearen einwertigen C₆- bis C₁₂-Alkanolen (z. B. 2-Propylheptylacrylat oder 2-Ethylhexylacrylat) sowie Mono- und Diester aus Maleinsäure und einwertigen C₄- bis C₁₀-Alkanolen in Betracht. Dabei gilt auch für alle vorgenannten Extraktionsmittel, dass diejenigen unter ihnen, die bei Normaldruck einen Siedepunkt oberhalb von 150 °C bzw. oberhalb von 160 °C, oder oberhalb von 170 °C, oder oberhalb von 180 °C, oder oberhalb von 190 °C aufweisen, erfindungsgemäß bevorzugt werden.

In der Regel wird das zu extrahierende Sauerwasser neben Acrylsäure und Wasser als weiteren Bestandteil (in der Regel in Gew.-% bezogen auf die Gesamtmenge des Sauerwassers der drittgrößte Bestandteil) Essigsäure enthalten. Je nach Art und Weise der durchgeführten Partialoxidation (ausgewähltem Katalysator, Wasserdampfgehalt des Reaktionsgasgemischs, Temperatur der Partialoxidation) kann das Sauerwasser bis zu 10 Gew.-%, bzw. bis zu 5 Gew.-% (häufig 2 bis 8 Gew.-%) oder mehr an Essigsäure enthalten. Häufig enthält das Sauerwasser etwa den 2-fachen Gewichtsanteil an Acrylsäure bezogen auf den Gewichtsanteil an Essigsäure. Die Gehalte der anderen möglichen sauren Nebenkomponenten liegen normalerweise signifikant tiefer. Erfindungsgemäß bevorzugt sind daher jene Extraktionsmittel, die die Acrylsäure im Vergleich zur Essigsäure bevorzugt aufnehmen. Zu diesen Extraktionsmitteln zählt im besonderen der Phthalsäurediethylester.

Erfindungsgemäß vorteilhaft ist es auch, wenn das Extraktionsmittel sich unter den Extraktionsbedingungen nicht mit Wasser umsetzt und in Wasser nur eine geringe Löslichkeit aufweist. So ist z. B. der Phthalsäurediethylester besonders hydrolysestabil. Ein weiterer Vorteil des Phthalsäurediethylesters ist sein bei Normaldruck (1 atm) vergleichsweise hoher Siedepunkt, der erfindungsgemäß vorteilhaft für zu verwendende Extraktionsmittel (organische Lösungsmittel) ≥ 200°C, besser ≥ 225°C und noch besser ≥ 250°C beträgt.

Zusätzlich weist er eine vergleichsweise geringe Löslichkeit in Wasser auf (dies mindert auch die Extraktionsmittelverluste). In der Regel fällt das Sauerwasser bei der erfindungsgemäß durchzuführenden fraktionierenden Kondensation des Produktgasgemischs mit einer Temperatur von 50 bis 80°C, vorzugsweise 60 bis 70°C an. D. h., mit dieser Temperatur wird es normalerweise über den zweiten Flüssigphasenabzug (vorzugsweise einen Seitenabzug) entnommen (je tiefer die Temperatur, desto geringer ist der Bedarf an Polymerisationsinhibitor; in günstigen Fällen bedarf es nicht des separaten Zusatzes eines solchen ins Sauerwasser, Extraktionsmittel, Raffinat und/oder Extrakt). Anwendungstechnisch zweckmäßig wird man daher auch die Extraktion in diesem Temperaturbereich durchführen. D. h., erfindungsgemäß vorteilhaft wird man das Sauerwasser im Wesentlichen mit seiner vorgenannten Temperatur in die Extraktionseinheit, vorzugsweise eine Extraktionskolonne (besonders bevorzugt eine Packungskolonne, vorteilhaft Montz-Pak B1-350) führen. Mit Vorteil erfolgt die Zufuhr von unten in die Extraktionskolonne und von oben wird das spezifisch schwerere Extraktionsmittel (mit Vorteil Diethylphthalat) aufgegeben. Üblicherweise wird die Temperatur des aufgegebenen Extraktionsmittels von jener des zugeführten Sauerwassers nicht sehr verschieden sein. In typischer Weise beträgt der Betrag dieses Temperaturunterschieds ≥ 0°C und ≤ 20°C, vorzugsweise ≥ 0°C und ≤ 15°C sowie vielfach ≥ 0°C und ≤ 10°C. Der Druck des der Kondensationskolonne entnommenen Sauerwassers beträgt an der Entnahmestelle erfindungsgemäß typisch > 1 bis 1,5 bar, häufig 2 bar. Das entnommene Sauerwasser wird mittels einer Pumpe in die Extraktionskolonne geführt. Der Förderdruck kann z. B. 2 bis 6 bar betragen. Der Arbeitsdruck in der Extraktionskolonne wird erfindungsgemäß so gewählt, dass es keiner zusätzlichen Pumpe bedarf, um das organische Extrakt in die erste Strippkolonne zu fördern. Grundsätzlich kann die Sauerwasserextraktion aber auch bei höheren oder tieferen Temperaturen sowie bei höheren oder niedrigeren Drucken durchgeführt werden. Bei der Inbetriebnahme einer Extraktionskolonne wird man anwendungstechnisch zweckmäßig so vorgehen, dass man die Extraktionskolonne zunächst mit Sauerwasser füllt und anschließend, wie bereits beschrieben, das organische Extraktionsmittel vorteilhaft am Kopf der Extraktionskolonne tropfenförmig aufgibt. Die Zufuhr des Sauerwassers (der bevorzugt kontinuierlichen Phase) kann prinzipiell unmittelbar über einen entsprechenden Zufuhrstutzen erfolgen. Grundsätzlich kann das Sauerwasser aber auch über ein (oder mehrere) Durchtrittsöffnungen in seiner Wandung aufweisendes Zufuhrrohr (Durchmesser der Durchtrittsöffnungen liegen bei typisch 5 bis 10 mm) zugeführt werden.

Das Verhältnis V der der Extraktionskolonne zugeführten Mengenströme an organischem Extraktionsmittel (E; in kg/h) und saurem Wasser (S; in kg/h), d. h., E:S, kann beim erfindungsgemäßen Verfahren 0,05 bis 20, vorzugsweise 0,1 bis 10, besser 0,8 bis 1,2 und besonders bevorzugt 1:1 betragen.

Das an Acrylsäure ausgelaugte (extrahierte, abgereicherte) Sauerwasser wird normalerweise seiner Entsorgung zugeführt (z. B. verbrannt oder in die Kläranlage gefahren). Es verlässt die Extraktionskolonne erfindungsgemäß typisch an deren Kopf (als Raffinat), während das organische, die Acrylsäure enthaltene Extrakt die Extraktionskolonne in typischer Weise unten verlässt.

Die Abtrennung der Acrylsäure aus dem organischen Extrakt, dessen Entnahmetemperatur aus der Extraktionskolonne im Wesentlichen der Zufuhrtemperatur des Sauerwassers in die Extraktionskolonne entspricht, kann grundsätzlich unter Anwendung unterschiedlicher thermischer Trennverfahren oder auch unter Anwendung von Kombinationen solcher thermischer Trennverfahren vorgenommen werden.

Eine geeignete Abtrennvariante ist die kristallisative Abtrennung. Dabei kommen alle Kristallisationsverfahren in Betracht, die in der DE-A 19838845 und in der DE-A 10 2005 015 637 empfohlen werden.

Im Folgenden werden Ausführungsbeispiele der erfindungsgemäßen Extraktionskolonne und Ausführungsbeispiele des erfindungsgemäßen Verfahrens mit Bezug zu den Zeichnungen erläutert.
- Fig. 1: zeigt eine schematische Ansicht der Extraktionskolonne gemäß einem Ausführungsbeispiel der Erfindung,
- Fig. 2: zeigt einen Querschnitt der Extraktionskolonne gemäß dem Ausführungsbeispiel der Erfindung,
- Fig. 3: zeigt einen Querschnitt einer Extraktionskolonne eines anderen Ausführungsbeispiels der Erfindung,
- Fig. 4: zeigt einen Querschnitt einer Extraktionskolonne gemäß einem noch weiteren Ausführungsbeispiel der Erfindung,
- Fig. 5: zeigt einen Teil eines Trennblechs einer Trenneinrichtung gemäß den Ausführungsbeispielen der Erfindung,
- Fig. 6: zeigt eine Trenneinrichtung gemäß dem ersten Ausführungsbeispiel der Erfindung,
- Fig. 7: zeigt die Aufnahme der Trenneinrichtung der Fig. 6 in einen Kolonnenkörper gemäß einem Ausführungsbeispiel der Erfindung,
- Fig. 8: zeigt die Verbindung der Trenneinrichtung mit dem Kolonnenkörper im Detail,
- Fig. 9: zeigt eine Einzelpackung zur Aufnahme in die Bereiche einer Extraktionskolonne gemäß Ausführungsbeispielen der Erfindung und
- Fig. 10: zeigt schematisch eine mit Packungen gefüllte Extraktionskolonne gemäß Ausführungsbeispielen der Erfindung.

Das im Folgenden beschriebene Ausführungsbeispiel betrifft die Extraktion von Acrylsäure aus Sauerwasser mittels eines organischen Lösungsmittels. Das Sauerwasser wurde dabei einer Kondensationskolonne zur Herstellung von Acrylsäure bei einem Seitenabzug entnommen. Es wird jedoch darauf hingewiesen, dass auch andere Bestandteile aus einem zu extrahierenden Fluid, insbesondere einer Extraktionsflüssigkeit, mittels eines Extraktionsmittels auf entsprechende Weise in ein Extraktgemisch überführt werden können.

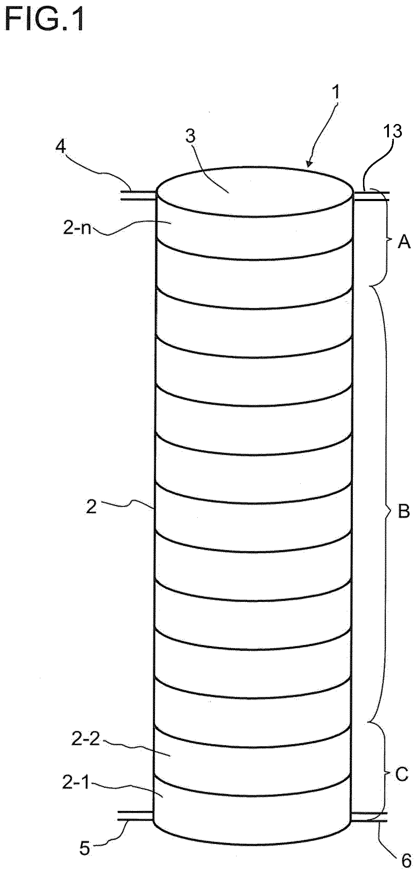

In Fig. 1 ist die Extraktionskolonne 1 schematisch dargestellt. Sie umfasst einen zylindrischen Kolonnenkörper 2, dessen Achse vertikal ausgerichtet ist. Bei dem Kolonnenkörper 2 handelt es sich im Wesentlichen um einen Hohlzylinder. Das heißt, der Mantel des Kolonnenkörpers 2 bildet einen Kolonnenhohlraum 3. Der Kolonnenkörper 2 ist aus Edelstahl gefertigt. Typische Wanddicken betragen 5 mm bis 20 mm. Nach außen ist die Extraktionskolonne 1 normalerweise in herkömmlicher Weise thermisch isoliert. Die Höhe der Extraktionskolonne 1 ist 40 m.

Für eine einfachere Montage des Kolonnenkörpers 2 ist dieser in Segmente 2-1, 2-2, ...., 2-n unterteilt. Bei der Montage des Kolonnenkörpers 2 werden die Segmente 2-1 bis 2-n nacheinander aufeinander gesetzt und dicht miteinander verbunden.

In vertikaler Richtung ist die Extraktionskolonne 1 in drei Bereiche unterteilt: Der obere Bereich wird als Kolonnenkopf A bezeichnet. Beim Kolonnenkopf A ist ein erster Zulauf 4 vorgesehen, über welchen beispielsweise das Extraktionsmittel in den Kolonnenhohlraum 3 eingeleitet werden kann. Der erste Zulauf 4 ist zweckmäßigerweise mit einem Rohrverteiler verbunden, über welchen das Extraktionsmittel gleichmäßig über den Querschnitt des Kolonnenkörpers 2 verteilt werden kann.

Beim Kolonnenkopf A ist außerdem ein Ablauf 13 vorgesehen, über welchen beispielsweise das Raffinat aus dem Kolonnenhohlraum 3 herausgeleitet werden kann.

Unterhalb des Kolonnenkopfes A ist ein Bereich B gebildet, bei dem im Kolonnenhohlraum 3 eine Trenneinrichtung 7 angeordnet ist, wie es später im Detail erläutert wird. Unterhalb des Bereichs B ist der Kolonnensumpf C gebildet. Beim Kolonnensumpf C befindet sich ein zweiter Zulauf 5, über den beispielsweise die zu extrahierende Flüssigkeit, d. h. im vorliegenden Fall das Sauerwasser, in den Kolonnenhohlraum 3 eingeleitet werden kann. Ferner befindet sich im Kolonnensumpf C ein Ablauf 6 für das Extraktgemisch.

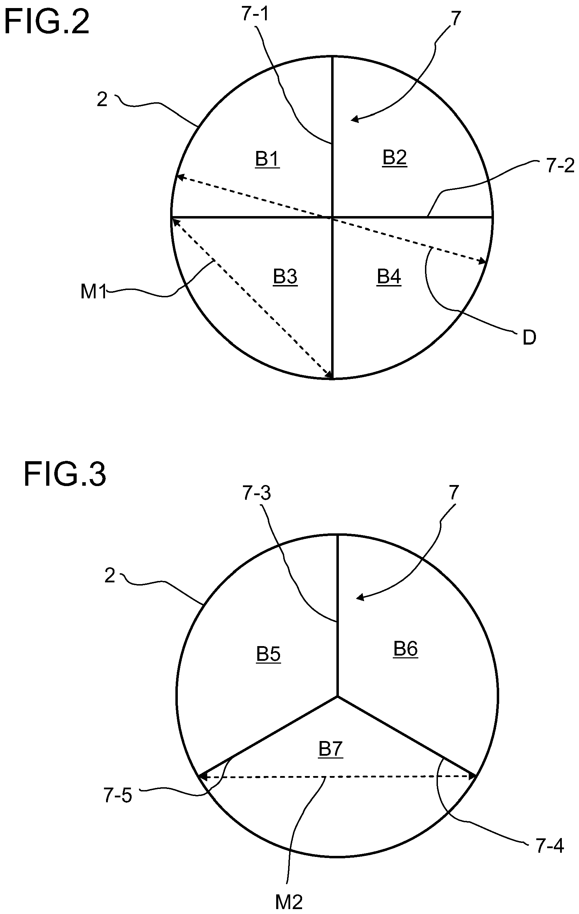

In Fig. 2 ist der horizontale Querschnitt der Extraktionskolonne 1 im Bereich B gezeigt, in dem die Trenneinrichtung 7 angeordnet ist. Über den gesamten Bereich B entspricht der Innendurchmesser D des Kolonnenkörpers 2 der horizontalen Maximalerstreckung des Kolonnenhohlraums 3. Diese horizontale Maximalerstreckung bezeichnet den größten Abstand zwischen zwei beliebigen Punkten der inneren Oberfläche des Kolonnenkörpers 2.

Im Bereich B des Kolonnenhohlraums 3 ist ferner eine Trenneinrichtung 7 angeordnet. Bei dem in Fig. 2 gezeigten Beispiel umfasst die Trenneinrichtung 7 zwei senkrecht zueinander angeordnete Trennbleche 7-1 und 7-2. Beide Trennbleche 7-1 und 7-2 sind in der Mitte des Kolonnenhohlraums 3, d. h. zusammenfallend mit der Achse des Kolonnenkörpers 2, miteinander verbunden, so dass sie senkrecht aufeinander stehen. Die Trennbleche 7-1 und 7-2 erstrecken sich horizontal bis zur Innenwand des Kolonnenkörpers 2 und schließen dort dicht ab. Die Trenneinrichtung 7 unterteilt den Kolonnenhohlraum 3 somit in vier identische vertikal ausgerichtete und horizontal getrennte Bereiche B1, B2, B3 und B4. Innerhalb dieser Bereiche B1 bis B4 kann die leichtere Phase, d. h. im vorliegenden Fall das Sauerwasser, aufsteigen und die schwerere Phase, d. h. im vorliegenden Fall das Extraktionsmittel, absteigen. Im Bereich B der Extraktionskolonne 1 ist jedoch ein Stoffaustausch zwischen den Bereichen B1 bis B4 nicht möglich.

Die Bereiche B1 bis B4 zeichnen sich dadurch aus, dass die horizontale Maximalerstreckung M1 jedes Bereichs D1 bis D4 im Bereich B der Extraktionskolonne 1 kleiner ist als die horizontale maximale Erstreckung D des Kolonnenkörpers 2. Bei dem in Fig. 2 gezeigten Beispiel ist das Verhältnis der horizontalen Maximalerstreckung M1 der Bereiche B1 bis B4 zu der horizontalen Maximalerstreckung D des Kolonnenhohlraums 3 gleich 1/√2, d. h. etwa 0,7. Durch die Trenneinrichtung 7 wurde somit die horizontale Maximalerstreckung innerhalb des Kolonnenkörpers 2 um etwa 30 % verringert.

In Fig. 3 ist ein anderes Beispiel einer Trenneinrichtung 7 gezeigt. In diesem Fall besteht die Trenneinrichtung 7 aus drei Trennblechen 7-3, 7-4 und 7-5. Diese sind in der Mitte miteinander so verbunden, dass sie einen Winkel von 120° einschließen. Von der Mitte erstrecken sie sich horizontal bis zur Innenwand des Kolonnenkörpers 2. Auf diese Weise werden drei vertikal ausgerichtete und horizontal getrennte Bereiche B5, B6 und B7 gebildet. Zwischen diesen Bereichen B5 bis B7 ist kein Stoffaustausch möglich.

Bei dem in Fig. 3 gezeigten Beispiel ist das Verhältnis der horizontalen Maximalerstreckung M2 der Bereiche D5 bis D7 zu der horizontalen Maximalerstreckung D des Kolonnenhohlraums 3 gleich cos 30°, d. h. etwa 0,86. Auch in diesem Fall wird somit die horizontale Maximalerstreckung des Kolonnenhohlraums 3 wesentlich verringert.

Die Bereiche B1 bis B4 des Beispiels gemäß Fig. 2 und die Bereiche B5 bis B7 des Beispiels gemäß Fig. 3 zeichnen sich außerdem dadurch aus, dass die Bereiche jeweils die identische Geometrie haben und dasselbe Volumen einschließen. Hierdurch wird gewährleistet, dass innerhalb der Bereiche B1 bis B4 bzw. der Bereiche B5 bis B7 jeweils die gleichen Bedingungen für die Extraktion herrschen werden.

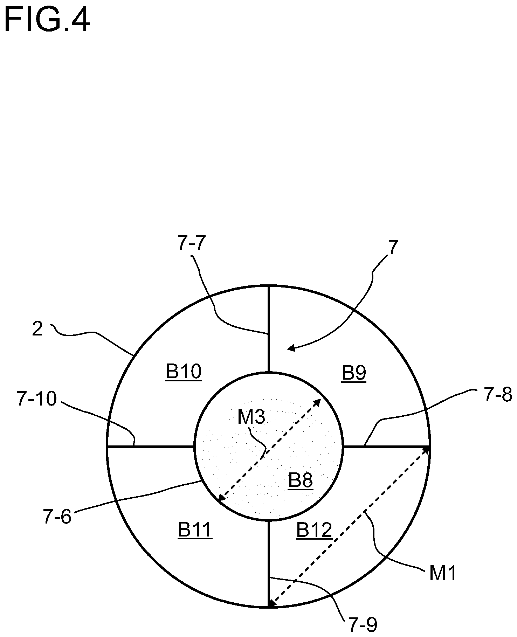

In Fig. 4 ist ein weiteres Beispiel für eine Trenneinrichtung 7 gezeigt, die im Bereich B der Extraktionskolonne 1 angeordnet ist. In diesem Fall umfasst die Trenneinrichtung 7 ein im Querschnitt kreisförmiges Trennblech 7-6, welches konzentrisch zu dem Kolonnenkörper 2 angeordnet ist. Auf diese Weise grenzt das Trennblech 7-6 einen vertikal ausgerichteten kreiszylindrischen Bereich B8 ab. Der Durchmesser des Kreises, d. h. die horizontale Maximalerstreckung M3 des Bereichs B8, ist dabei kleiner als die horizontale Maximalerstreckung D des Kolonnenhohlraums 3.

Von der Außenseite des Trennblechs 7-6 erstrecken sich vier weitere Trennbleche 7-7 bis 7-10 radial nach außen bis zur Innenwand des Kolonnenkörpers 2. Dabei sind die Trennbleche 7-7 und 7-9 auf gegenüberliegenden Seiten des Trennblechs 7-6 angeordnet. Gleichermaßen sind die Trennbleche 7-8 und 7-10 auf gegenüberliegenden Seiten des Trennblechs 7-6 angeordnet. Die Trennbleche 7-7 und 7-9 sind auf einer ersten gedachten Gerade angeordnet, die Trennbleche 7-8 und 7-10 sind auf einer zweiten gedachten Gerade angeordnet. Dabei steht die erste gedachte Gerade senkrecht auf der zweiten gedachten Geraden. Durch die Trennbleche 7-7 bis 7-10 und das Trennblech 7-6 sowie den Kolonnenkörper 2 werden vier weitere Bereiche B9, B10, B11 und B12 abgegrenzt.

Die Querschnittsfläche der Bereiche B9 bis B12 ist identisch. Sie unterscheidet sich jedoch von der scheibenförmigen Querschnittsfläche des Bereichs B8. Der Durchmesser M3 des Bereichs B8 ist jedoch so gewählt, dass die Querschnittsflächen der Bereiche B8 bis B12 gleich groß sind, so dass die Bereiche B8 bis B12 dieselben Volumina umfassen. Dies bedeutet, dass das Verhältnis des Durchmessers D des Kolonnenhohlraums zu dem Durchmesser M3 des Bereichs D8 gleich √5 ist.

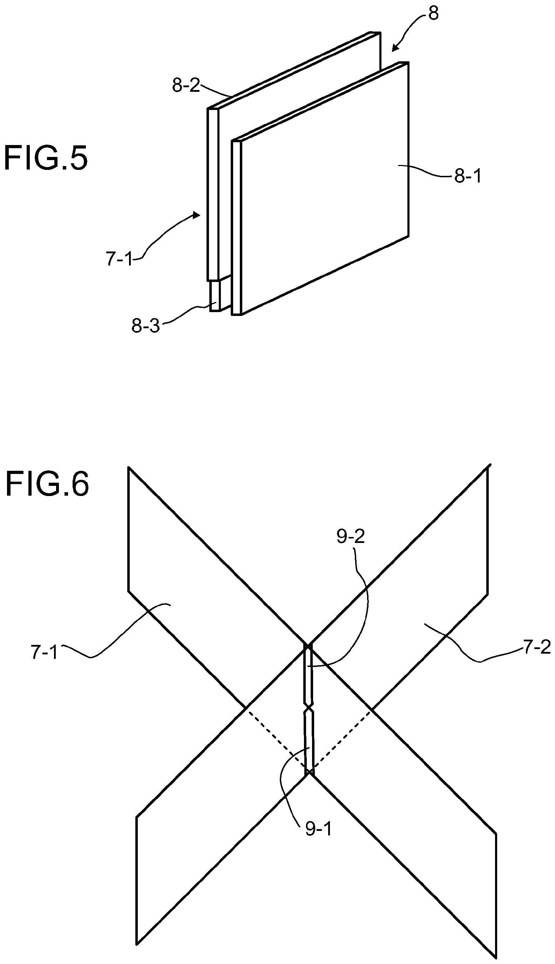

Mit Bezug zur Fig. 5 wird die Ausbildung des Trennblechs 7-1 im Detail beschrieben. Alle anderen Trennbleche 7-2 bis 7-5 und 7-7 bis 7-10, welche bis an die Innenwand des Mantels des Kolonnenkörpers 2 reichen, können entsprechend aufgebaut sein. Das Trennblech 7-1, das in Fig. 5 in einem Ausschnitt gezeigt ist, besteht aus mehreren Schichten 8. In Fig. 5 ist ein dreischichtiger Aufbau aus den Schichten 8-1, 8-2 und 8-3 gezeigt. Die Schichten sind flächig aneinander gefügt, wobei die mittlere Schicht 8-2 oben und an den Seiten, an denen das Trennblech 7-1 an die Innenwand des Kolonnenkörpers 2 angrenzt, vorsteht. Im unteren Bereich des Trennblechs 7-1 ist entsprechend eine Nut in der Mitte gebildet, so dass mehrere Trennbleche 7-1 aufeinander gesteckt werden können, wobei jeweils die mittlere Schicht 8-2 seitlich vorsteht.

Wie in Fig. 6 gezeigt weisen die Trennbleche 7-1 und 7-2, welche zu der in Fig. 2 gezeigten Trenneinrichtung 7 zusammengefügt werden, in der Mitte Schlitze 9-1 und 9-2 auf. Der Schlitz 9-1 des Trennblechs 7-1 verläuft dabei von unten nach oben, und der Schlitz 9-2 des Trennblechs 7-2 verläuft von oben nach unten, so dass die Trennbleche 7-1 und 7-2 kreuzweise ineinander gesteckt werden können. Die zusammengesetzten Trennbleche 7-1 und 7-2, wie sie in Fig. 6 gezeigt sind, bilden dabei ein Vertikalsegment der Trenneinrichtung 7. Mehrere aufeinander gesteckte Vertikalsegmente ergeben die vollständige Trenneinrichtung 7, wie sie in Fig. 2 gezeigt ist.

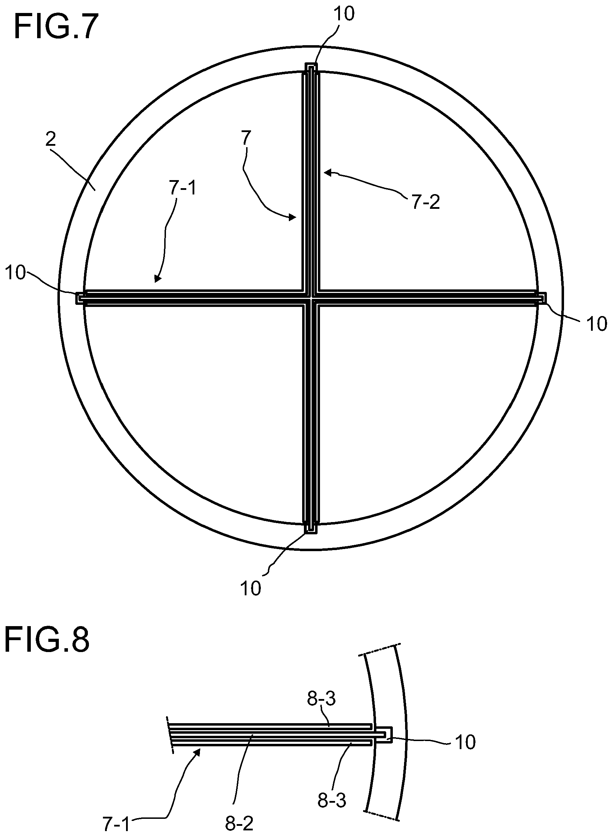

Mit Bezug zu den Figuren 7 und 8 wird die Kopplung der Trenneinrichtung 7 mit der Innenwand des Kolonnenkörpers 2 erläutert. Für die verdrehsichere Aufnahme der Trenneinrichtung 7 bei der Innenwand des Kolonnenkörpers 2 sind in dieser Innenwand Nuten 10 gebildet. Die Nuten 10 sind jeweils bei der Position bei der Innenwand des Kolonnenkörpers 2 angeordnet, bei welcher die Trenneinrichtung 7 einen Bereich dicht abschließt. Die Trennbleche 7-1 und 7-2 sind so dimensioniert, dass die Quererstreckung der Schichten 8-1 und 8-3 im Wesentlichen dem Innendurchmesser D des Kolonnenkörpers 2 entspricht. Die mittlere Schicht 8-2 steht nun soweit hervor, dass sie exakt in die Nuten 10 eingreift. In den Figuren 7 und 8 ist dies schematisch dargestellt. Im Idealfall werden die Bereiche B1 bis B4 durch den Anschluss der Stirnflächen der Schichten 8-1 bis 8-2 der Trennbleche 7-1 und 7-2 an der Innenwand des Kolonnenkörpers 2 und der Stirnfläche der Schicht 8-3 in der entsprechenden Nut 10 abgedichtet. Die ineinander gesteckten Trennbleche 7-1 und 7-2 können auf diese Weise von oben in ein Segment 2-1 bis 2-n des Kolonnenkörpers 2 eingesteckt werden.

Die von der Trenneinrichtung 7 abgetrennten Bereiche des Kolonnenhohlraums werden von trennwirksamen Einbauten ausgefüllt. Im vorliegenden Ausführungsbeispiel wurden als Einbauten strukturierte Packungen der Ausführung B1-350 flach und gelocht der Firma Montz oder dieselbe Ausführung SMV 350 der Firma Sulzer verwendet.

Die Geometrie der Packungen entspricht der Geometrie der von der Trenneinrichtung 7 abgetrennten Bereiche. In vertikaler Richtung sind die Packungen jedoch unterteilt. Es werden mehrere identische Einzelpackungen aufeinander gesetzt.

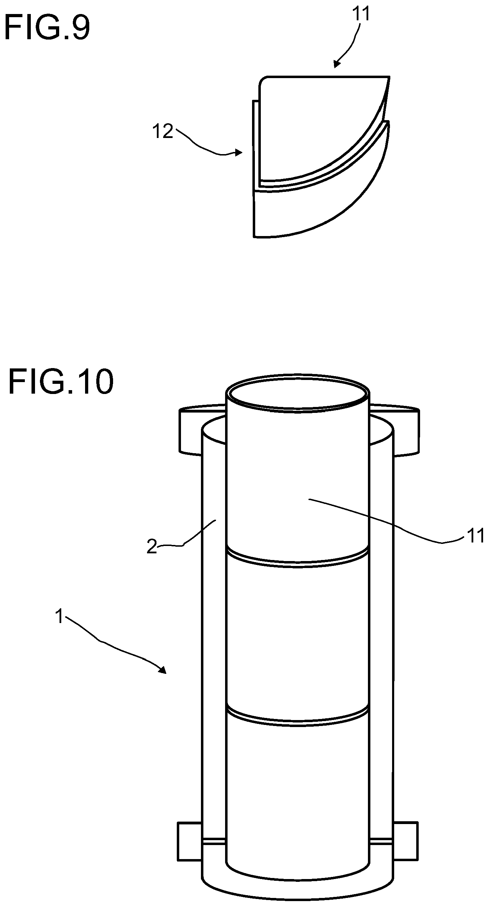

In Fig. 9 ist eine solche Einzelpackung 11 gezeigt. Sie ist für die in Fig. 2 gezeigte Trenneinrichtung 7 ausgebildet. Die Querschnittsfläche dieser Einzelpackung 11 entspricht dabei genau der Querschnittsfläche der Bereiche B1 bis B4. Für alle Bereiche B1 bis B4 können somit dieselben Einzelpackungen 11 verwendet werden. Um Toleranzen bei der Herstellung der Einzelpackungen 11 auszugleichen, ist die Einzelpackung 11 mit einem Metallgewebeband 12 umwickelt.

Die Extraktionskolonne 1 wird somit wie folgt montiert:
Zunächst wird ein Teil des Kolonnenkörpers 2 aufgebaut. Hierfür kann das unterste Segment 2-1 des Kolonnenkörpers 2 aufgestellt werden. Gegebenenfalls können aber auch mehrere Segmente 2-1 bis 2-m bis zu einer bestimmten Höhe aufeinander montiert werden. Die Höhe wird dabei so gewählt, dass ein Monteur von oben einem Monteur am Boden des Teils des Kolonnenkörpers 2 sicher Trennbleche 7-1 und 7-2 und Packungen 11 herunterlassen kann. Es soll vermieden werden, dass herunterfallende Teile einen Monteur am Boden des Kolonnenkörpers 2 ernsthaft verletzen können. Beispielsweise wird der Kolonnenkörper 2 zunächst bis zu einer Höhe von 10 m aufgebaut.

Das unterste Segment 2-1 umfasst dabei den zweiten Zulauf 5 sowie den Ablauf 6 sowie gegebenenfalls Rohrverteiler im Inneren des Kolonnenkörpers 2. Wenn eine Packung im Kolonnensumpf C des Kolonnenkörpers 2 angeordnet werden soll, wird zunächst diese Packung im Kolonnensumpf C montiert. Die Anordnung einer Packung im Kolonnensumpf ist optional. Als Packung könnte hier ein Füllkörper wie die Pall-Ringe DN25 eingesetzt werden.

Anschließend wird ein Vertikalsegment der Trenneinrichtung 7 eingesetzt. Hierfür weisen die Segmente des Kolonnenkörpers 2 im Bereich B die Nuten 10 auf, wie sie in den Figuren 7 und 8 gezeigt sind.

Einem Monteur, der am Boden des Kolonnenkörpers 2 steht, werden die Trennbleche 7-1 und 7-2 einer Trenneinrichtung 7 heruntergelassen. Dieser setzt sie kreuzweise zusammen, wie es in Fig. 6 gezeigt ist, und befestigt die Trenneinrichtungen 7 in den Nuten 10 des Kolonnenkörpers 2. Daraufhin werden dem Monteur vier Einzelpackungen 11, wie sie in Fig. 9 gezeigt sind, heruntergelassen. Der Monteur setzt diese Einzelpackungen 11 in die vier Bereiche, welche durch die Trenneinrichtung 7 gebildet worden sind, ein. Anschließend werden dem Monteur weitere Trennbleche 7-1 und 7-2 heruntergelassen, welche er kreuzweise zusammensteckt und so auf die bereits montierte Trenneinrichtung 7 setzt, dass der nach oben herausstehende Teil der Schicht 8-2 der bereits montierten Trenneinrichtung 7 in die untere Nut der zu montierenden Trenneinrichtung 7 eingreift. Ferner greifen auch die nach außen vorstehenden Teile der mittleren Schicht 8-2 in die Nuten 10 ein. Anschließend werden erneut vier Einzelpackungen 11 auf die darunter liegenden Einzelpackungen 11 aufgesetzt. Auf diese Weise wird der montierte Teil des Kolonnenkörpers 2 nachfolgend mit Vertikalsegmenten der Trenneinrichtung 7 und mit Einzelpackungen 11 aufgefüllt.

Ist man am oberen Ende des Teils des Kolonnenkörpers 2 angelangt, wird ein weiterer Teil des Kolonnenkörpers 2 mittels der Einzelsegmente montiert. Anschließend werden auf gleiche Weise weitere Vertikalsegmente der Trenneinrichtung 7 und Einzelpackungen 11 in dem nach oben wachsenden Kolonnenkörper 2 montiert, bis der Kolonnenkörper 2 vollständig aufgebaut ist. Am Kolonnenkopf A werden keine Trenneinrichtungen 7 mehr montiert. Wie beim Kolonnensumpf C werden jedoch Packungen eingesetzt, welche sich über den gesamten Innendurchmesser des Mantels des Kolonnenkörpers erstrecken. In Fig. 10 ist der vollständig aufgebaute Kolonnenkörper 2 der Extraktionskolonne 1 gezeigt. Dabei kann das oberste Packungssegment etwas, z. B. 10 cm, über den Kolonnenkörper 2 hinausragen. Im Bereich des Kolonnenkopfes A können noch entsprechende Rohrverteiler montiert werden, welche mit dem ersten Zulauf 4 gekoppelt sind, um das Extraktionsmittel gleichmäßig über die Querschnittsfläche des Kolonnenhohlraums 3 zu verteilen und die darunterliegenden getrennten Bereiche B1 bis B4 bzw. B5 bis B7 bzw. B8 bis B12 zu beaufschlagen.

Im Folgenden wird ein Ausführungsbeispiel des erfindungsgemäßen Verfahrens beschrieben, welches mit der vorstehend beschriebenen Extraktionskolonne 1 ausgeführt wird.

Über den zweiten Zulauf 5 wird das zu extrahierende Sauerwasser unterhalb der untersten Packung 11 in die Extraktionskolonne 1 über einen Rohrverteiler zugeführt, welcher entsprechende Durchtrittsöffnungen aufweist.

Das nicht mit Inhibitor versetzte Sauerwasser weist z. B. folgende Gehalte auf:

| | |
|---|---|
| 2,36 Gew.-% | Formaldeyhd, |
| 83,12 Gew.-% | Wasser, |
| 3,98 Gew.-% | Essigsäure, |
| 9,70 Gew.-% | Acrylsäure, |
| 0,68 Gew.-% | Ameisensäure, und |
| 0,01 Gew.-% | Diacrylsäure. |

Die spezifische Masse des Sauerwassers beträgt 967,5 kg/m³.

Anschließend wird oberhalb der obersten Packung das Extraktionsmittel über den ersten Zulauf 4 und einen Rohrverteiler, der entsprechende Durchtrittsöffnungen (Bohrungen des Durchmessers 4 mm) aufweist, aufgegeben.

Das Extraktionsmittel weist z. B. folgende Gehalte auf:

| | |
|---|---|
| ≤ 0,5 Gew.-% | Acrylsäure, |
| ≤ 0,03 Gew.-% | Essigsäure, |
| ≤ 0,02 Gew.-% | Wasser, |

| | |
|---|---|
| ≤ 0,001 Gew.-% | Ameisensäure, |
| ≤ 0,0035 Gew.-% | Acrolein, |
| ≤ 0,0005 Gew.-% | Propionsäure, |
| ≤ 0,0001 Gew.-% | Furfurale, |
| ≤ 0,001 Gew.-% | Allylformiat, |
| 0,03 Gew.-% | MEHQ, |
| 0,0001 Gew.-% | Sauerstoff, und |
| ≥ 99,5 Gew.-% | Palatinol® A. |

Die spezifische Masse des Extraktionsmittels beträgt 1120 kg/m³.

Das Extraktgemisch weist einen gegenüber dem Extraktionsmittel erhöhten Anteil an Acrylsäure auf und das Raffinat weist einen gegenüber dem Sauerwasser verringerten Anteil an Acrylsäure auf.

Das Sauerwasser bildet die kontinuierliche Phase und das Extraktionsmittel bildet die tropfenförmig dispers verteilte Phase (Tropfendurchmesser im Bereich von 2 bis 5 mm liegend), die in der wässrigen Phase absteigt.

Am Kopf A der Extraktionskolonne 1 wird das Raffinat entnommen. Es weist einen gegenüber dem Sauerwasser reduzierten Gehalt an Acrylsäure auf. Es wird gemeinsam mit zu verbrennendem Restgas der Verbrennung zugeführt.

Dem Sumpf C der Extraktionskolonne 1 wird das Extraktgemisch entnommen. Es weist einen gegenüber dem Extraktionsmittel erhöhten Gehalt an Acrylsäure auf.

### Bezugszeichenliste

- 1: Extraktionskolonne
- 2: Kolonnenkörper
- 2-1 bis 2-n: Segmente des Kolonnenkörpers
- 3: Kolonnenhohlraum
- 4: erster Zulauf
- 5: zweiter Zulauf
- 6: Ablauf
- 7: Trenneinrichtung
- 7-1 bis 7-10: Trennbleche
- 8, 8-1 bis 8-3: Schichten
- 9, 9-1, 9-2: Schlitze
- 10: Nuten
- 11: Packung; Einzelpackung
- 12: Gewebeband aus Metall
- 13: Ablauf

## Patentansprüche

1. Extraktionskolonne (1) mit
einem zumindest abschnittsweise zylindrischen, vertikal ausgerichteten Kolonnenkörper (2), der einen Kolonnenhohlraum (3) bildet, der eine horizontale Maximalerstreckung aufweist, wobei in dem Kolonnenkörper (2) mindestens ein erster Zulauf (4) für ein Extraktionsmittel, mindestens ein zweiter Zulauf (5) für das zu extrahierende Fluid und mindestens ein Ablauf (6) für das Extraktgemisch und mindestens ein Ablauf (13) für das Raffinat ausgebildet ist, **dadurch gekennzeichnet, dass**
in dem Kolonnenhohlraum (3) eine vertikal ausgerichtete Trenneinrichtung (7) angeordnet ist, die den Kolonnenhohlraum (3) in mehrere vertikal ausgerichtete und horizontal getrennte Bereiche (B1 bis B4; B5 bis B7; B8 bis B12) unterteilt, wobei die horizontale Maximalerstreckung (M1; M2; M3) jedes Bereichs (B1 bis B4; B5 bis B7; B8 bis B12) bei jedem Horizontalschnitt durch den Kolonnenkörper (2), der die Trenneinrichtung (7) schneidet, kleiner als die horizontale Maximalerstreckung (D) des Kolonnenhohlraums (3) ist,
die Bereiche (B1 bis B4; B5 bis B7; B8 bis B12) bei ihren oberen Enden in einen gemeinsamen Kolonnenkopf (A) und bei ihren unteren Enden in einen gemeinsamen Kolonnensumpf (C) münden, wobei im Bereich des Kolonnenkopfes (A) und im Bereich des Kolonnensumpfes (C) die horizontale Erstreckung des Kolonnenhohlraums (3) nicht durch die Trenneinrichtung (7) unterteilt ist und beim Kolonnenkopf (A) einer der Zuläufe (4, 5) und beim Kolonnensumpf (C) der andere der Zuläufe (5, 4) angeordnet ist.

2. Extraktionskolonne (1) nach Anspruch 1, **dadurch gekennzeichnet, dass** der Kolonnenkörper (2) bei einem horizontalen Schnitt durch den Kolonnenkörper (2) kreisförmig ist, und dass die Trenneinrichtung (7) den Kolonnenhohlraum (3) in vier vertikal ausgerichtete und horizontal getrennte Bereiche (B1 bis B4) unterteilt.

3. Extraktionskolonne (1) nach einem der vorhergehenden Ansprüche, **dadurch gekennzeichnet, dass** die von der Trenneinrichtung (7) gebildeten Bereiche (B1 bis B4; B5 bis B7; B8 bis B12) gleich groß sind.

4. Extraktionskolonne (1) nach einem der vorhergehenden Ansprüche, **dadurch gekennzeichnet, dass** die horizontale Maximalerstreckung (D) des Kolonnenhohlraums (3) größer oder gleich 800 mm ist, insbesondere größer oder gleich 1000 mm ist.

5. Extraktionskolonne (1) nach einem der vorhergehenden Ansprüche, **dadurch gekennzeichnet, dass** in dem Kolonnenhohlraum (3) trennwirksame Einbauten (11) angeordnet sind.

6. Extraktionskolonne (1) nach Anspruch 5, **dadurch gekennzeichnet, dass** die trennwirksamen Einbauten (11) für die durch die Trenneinrichtung (7) gebildeten Bereiche (B1 bis B4; B5 bis B7; B8 bis B12) separat voneinander vorgesehen sind.

7. Extraktionskolonne (1) nach Anspruch 5 oder 6, **dadurch gekennzeichnet, dass** die trennwirksamen Einbauten so ausgebildet sind, dass sie Flüssigkeitstropfen bilden.

8. Extraktionskolonne (1) nach einem der vorhergehenden Ansprüche, **dadurch gekennzeichnet, dass** die Trenneinrichtung (7) in Vertikalsegmente unterteilt ist, die in dem Kolonnenhohlraum (3) aufeinander gesetzt sind.

9. Extraktionskolonne (1) nach einem der Ansprüche 5 bis 8, **dadurch gekennzeichnet, dass** die trennwirksamen Einbauten in den von der Trenneinrichtung (7) gebildeten Bereichen (B1 bis B4; B5 bis B7) aus identischen Einzelpackungen (11) gebildet sind.

10. Extraktionskolonne (1) nach einem der vorhergehenden Ansprüche, **dadurch gekennzeichnet, dass** auf der Innenseite des Kolonnenkörpers (2) zumindest eine vertikal ausgerichtete Nut (10) gebildet ist, in welche die Trenneinrichtung (7) eingreift.

11. Extraktionskolonne (1) nach Anspruch 10, **dadurch gekennzeichnet, dass** die Trenneinrichtung (7) zumindest drei Schichten (8) umfasst, wobei die mittlere Schicht (8-2) gegenüber den äußeren Schichten (8-1, 8-3) vorsteht und in die zumindest eine Nut (10) eingreift.

12. Verfahren zum Extrahieren eines Bestandteils aus einem Fluid mittels einer Extraktionskolonne (1) gemäß Anspruch 1, die einen zylindrischen, vertikal ausgerichteten Kolonnenkörper (2) aufweist, der einen Kolonnenhohlraum (3) bildet, der eine horizontale Maximalerstreckung (D) aufweist, und der in vertikaler Richtung in einen Kolonnenkopf (A), einen Bereich (B), bei dem eine vertikal ausgerichtete Trenneinrichtung (7) angeordnet ist, und einen Kolonnensumpf (C) unterteilt ist, wobei beim Kolonnenkopf (A) ein erster Zulauf (4) und beim Kolonnensumpf (C) ein zweiter Zulauf (5) vorgesehen ist, wobei bei dem Verfahren
das zu extrahierende Fluid über einen der Zuläufe (4, 5) in den Kolonnenhohlraum (3) und dann in mehrere vertikal ausgerichtete und horizontal getrennte Bereiche (B1 bis B4; B5 bis B7; B8 bis B12) eingeleitet wird, die in dem Kolonnenhohlraum (3) von der vertikal ausgerichteten Trenneinrichtung (7) gebildet werden, die den Kolonnenhohlraum (3) in die Bereiche (B1 bis B4; B5 bis B7; B8 bis B12) unterteilt, wobei die horizontale Maximalerstreckung (M1; M2; M3) jedes Bereichs (B1 bis B4; B5 bis B7; B8 bis B12) bei jedem Horizontalschnitt durch den Kolonnenkörper (2), der die Trenneinrichtung (7) schneidet, kleiner als die horizontale Maximalerstreckung (D) des Kolonnenhohlraums (3) ist, wobei die Bereiche (B1 bis B4; B5 bis B7; B8 bis B12) bei ihren oberen Enden in den gemeinsamen Kolonnenkopf (A) und bei ihren unteren Enden in den gemeinsamen Kolonnensumpf (C) münden und wobei im Bereich des Kolonnenkopfes (A) und im Bereich des Kolonnensumpfes (C) die horizontale Erstreckung des Kolonnenhohlraums (3) nicht durch die Trenneinrichtung (7) unterteilt ist,
ein Extraktionsmittel über den anderen der Zuläufe (4, 5) in den Kolonnenhohlraum (3) und dann in die Bereiche (B1 bis B4; B5 bis B7; B8 bis B12) des Kolonnenhohlraums (3) eingeleitet wird, und
der Bestandteil des zu extrahierenden Fluids in den Bereichen (B1 bis B4; B5 bis B7; B8 bis B12) des Kolonnenhohlraums (3) von dem Extraktionsmittel aufgenommen wird und als Extraktgemisch entnommen wird.

13. Verfahren nach Anspruch 12, **dadurch gekennzeichnet, dass** bei den Bereichen (B1 bis B4; B5 bis B7; B8 bis B12) das Verhältnis des Extraktionsmittels zu dem zu extrahierenden Fluid über den Querschnitt der Extraktionskolonne (1) im Wesentlichen gleich ist.

14. Verfahren nach einem der Ansprüche 12 oder 13, **dadurch gekennzeichnet, dass** bei den Bereichen (B1 bis B4; B5 bis B7; B8 bis B12) die Zusammensetzung des Extraktionsmittels und/oder die Zusammensetzung des zu extrahierenden Fluids über den Querschnitt der Extraktionskolonne (1) im Wesentlichen gleich ist.

15. Verfahren nach einem der Ansprüche 12 bis 14, **dadurch gekennzeichnet, dass** das Extraktionsmittel ein organisches Lösungsmittel ist und das zu extrahierende Fluid Acrylsäure und Essigsäure enthaltendes Wasser ist.

## Claims

1. An extraction column (1) having
a vertically aligned column body (2) which is cylindrical at least in sections and forms a column cavity (3) having a horizontal maximum extent, with provision in the column body (2) of at least one first feed (4) for an extractant, at least one second feed (5) for the fluid to be extracted and at least one outlet (6) for the extract mixture and at least one outlet (13) for the raffinate,
wherein
a vertically aligned divider (7) arranged within the column cavity (3) subdivides the column cavity (3) into a plurality of vertically aligned and horizontally divided regions (B1 to B4; B5 to B7; B8 to B12), the horizontal maximum extent (M1; M2; M3) of each region (B1 to B4; B5 to B7; B8 to B12) at each horizontal section through the column body (2) cut by the divider (7) being less than the horizontal maximum extent (D) of the column cavity (3),
the regions (B1 to B4; B5 to B7; B8 to B12) open at their upper ends into a common column head (A) and at their lower ends into a common column bottom (C), where the horizontal extent of the column cavity (3) in the region of the column head (A) and in the region of the column bottom (C) is not subdivided by the divider (7), and one of the feeds (4, 5) is disposed in the column head (A) and the other of the feeds (5, 4) in the column bottom (C).

2. The extraction column (1) according to claim 1 or 2, wherein the column body (2) is circular at a horizontal section through the column body (2), and the divider (7) subdivides the column cavity (3) into four vertically aligned and horizontally divided regions (B1 to B4).

3. The extraction column (1) according to any of the preceding claims, wherein the regions (B1 to B4; B5 to B7; B8 to B12) formed by the divider (7) are of equal size.

4. The extraction column (1) according to any of the preceding claims, wherein the horizontal maximum extent (D) of the column cavity (3) is greater than or equal to 800 mm, especially greater than or equal to 1000 mm.

5. The extraction column (1) according to any of the preceding claims, wherein separating internals (11) are arranged within the column cavity (3).

6. The extraction column (1) according to claim 5, wherein the separating internals (11) for the regions (B1 to B4; B5 to B7; B8 to B12) formed by the divider (7) are provided separately from one another.

7. The extraction column (1) according to claim 5 or 6, wherein the separating internals are designed such that they form liquid droplets.

8. The extraction column (1) according to any of the preceding claims, wherein the divider (7) is subdivided into vertical segments placed one on top of another in the column cavity (3).

9. The extraction column (1) according to any of claims 5 to 8, wherein the separating internals in the regions (B1 to B4; B5 to B7) formed by the divider (7) are formed from identical individual packing elements (11).

10. The extraction column (1) according to any of the preceding claims, wherein at least one vertically aligned groove (10) in which the divider (7) engages is formed on the inside of the column body (2).

11. The extraction column (1) according to claim 10, wherein the divider (7) comprises at least three layers (8), and the middle layer (8-2) protrudes with respect to the outer layers (8-1, 8-3) and engages in the at least one groove (10).

12. A process for extracting a constituent from a fluid by means of an extraction column (1) according to claim 1, having a cylindrical, vertically aligned column body (2) which forms a column cavity (3) having a horizontal maximum extent (D), and which is subdivided in the vertical direction into a column head (A), a region (B) in which a vertically aligned divider (7) is disposed, and a column bottom (C), where a first feed (4) is provided in the column head (A) and a second feed (5) in the column bottom (C), wherein the process involves
introducing the fluid to be extracted via one of the feeds (4, 5) into the column cavity (3) and then into a plurality of vertically aligned and horizontally divided regions (B1 to B4; B5 to B7; B8 to B12) which are formed in the column cavity (3) by the vertically aligned divider (7) which subdivides the column cavity (3) into the regions (B1 to B4; B5 to B7; B8 to B12), the horizontal maximum extent (M1; M2; M3) of each region (B1 to B4; B5 to B7; B8 to B12) at each horizontal section through the column body (2) cut by the divider (7) being less than the horizontal maximum extent (D) of the column cavity (3), where the regions (B1 to B4; B5 to B7; B8 to B12) open at their upper ends into the common column head (A) and at their lower ends into the common column bottom (C), and where the horizontal extent of the column cavity (3) in the region of the column head (A) and in the region of the column bottom (C) is not subdivided by the divider (7),
introducing an extractant into the column cavity (3) via the other of the feeds (4, 5) and then into the regions (B1 to B4; B5 to B7; B8 to B12) of the column cavity (3), and
absorbing the constituent of the fluid to be extracted with the extractant in the regions (B1 to B4; B5 to B7; B8 to B12) of the column cavity (3) and withdrawing it as an extract mixture.

13. The process according to claim 12, wherein the ratio of the extractant to the fluid to be extracted in the regions (B1 to B4; B5 to B7; B8 to B12) is essentially equal over the cross section of the extraction column (1).

14. The process according to any of claims 12 and 13, wherein the composition of the extractant and/or the composition of the fluid to be extracted in the regions (B1 to B4; B5 to B7; B8 to B12) is essentially equal over the cross section of the extraction column (1).

15. The process according to any of claims 12 to 14, wherein the extractant is an organic solvent and the fluid to be extracted is water comprising acrylic acid and acetic acid.

## Revendications

1. Colonne d'extraction (1), comprenant :
un corps de colonne (2) cylindrique au moins en sections, orienté verticalement, qui forme une cavité de colonne (3), qui présente une extension maximale horizontale, au moins une première entrée (4) pour un agent d'extraction, au moins une deuxième entrée (5) pour le fluide à extraire et au moins une sortie (6) pour le mélange extrait et au moins une sortie (13) pour le raffinat étant formées dans le corps de colonne (2), **caractérisée en ce que**
un dispositif de séparation orienté verticalement (7) est agencé dans la cavité de colonne (3), qui divise la cavité de colonne (3) en plusieurs zones orientées verticalement et séparées horizontalement (B1 à B4 ; B5 à B7 ; B8 à B12), l'extension maximale horizontale (M1 ; M2 ; M3) de chaque zone (B1 à B4 ; B5 à B7 ; B8 à B12) au niveau de chaque coupe horizontale au travers du corps de colonne (2), qui coupe le dispositif de séparation (7), étant inférieure à l'extension maximale horizontale (D) de la cavité de colonne (3),
les zones (B1 à B4 ; B5 à B7 ; B8 à B12) débouchant à leurs extrémités supérieures dans une tête de colonne commune (A) et à leurs extrémités inférieures dans un fond de colonne commun (C), l'extension horizontale de la cavité de colonne (3) n'étant pas divisée par le dispositif de séparation (7) dans la zone de la tête de colonne (A) et dans la zone du fond de colonne (C), et une des entrées (4, 5) étant agencée dans la tête de colonne (A) et l'autre des entrées (5, 4) étant agencée dans le fond de colonne (C).

2. Colonne d'extraction (1) selon la revendication 1, **caractérisée en ce que** le corps de colonne (2) est circulaire au niveau d'une coupe horizontale au travers du corps de colonne (2), et **en ce que** le dispositif de séparation (7) divise la cavité de colonne (3) en quatre zones orientées verticalement et séparées horizontalement (B1 à B4).

3. Colonne d'extraction (1) selon l'une quelconque des revendications précédentes, **caractérisée en ce que** les zones (B1 à B4 ; B5 à B7 ; B8 à B12) formées par le dispositif de séparation (7) sont de même taille.

4. Colonne d'extraction (1) selon l'une quelconque des revendications précédentes, **caractérisée en ce que** l'extension maximale horizontale (D) de la cavité de colonne (3) est supérieure ou égale à 800 mm, notamment supérieure ou égale à 1 000 mm.

5. Colonne d'extraction (1) selon l'une quelconque des revendications précédentes, **caractérisée en ce que** des composants à activité de séparation (11) sont agencés dans la cavité de colonne (3).

6. Colonne d'extraction (1) selon la revendication 5, **caractérisée en ce que** les composants à activité de séparation (11) pour les zones (B1 à B4 ; B5 à B7 ; B8 à B12) formées par le dispositif de séparation (7) sont prévus séparément les uns des autres.

7. Colonne d'extraction (1) selon la revendication 5 ou 6, **caractérisée en ce que** les composants à activité de séparation sont configurés de telle sorte qu'ils forment des gouttes de liquide.

8. Colonne d'extraction (1) selon l'une quelconque des revendications précédentes, **caractérisée en ce que** le dispositif de séparation (7) est divisé en segments verticaux qui sont superposés dans la cavité de colonne (3) .

9. Colonne d'extraction (1) selon l'une quelconque des revendications 5 à 8, **caractérisée en ce que** les composants à effet de séparation dans les zones (B1 à B4 ; B5 à B7) formées par le dispositif de séparation (7) sont formés par des garnissages individuels identiques (11).

10. Colonne d'extraction (1) selon l'une quelconque des revendications précédentes, **caractérisée en ce qu'**au moins une rainure (10) orientée verticalement, avec laquelle le dispositif de séparation (7) entre en prise, est formée sur le côté intérieur du corps de colonne (2).

11. Colonne d'extraction (1) selon la revendication 10, **caractérisée en ce que** le dispositif de séparation (7) comprend au moins trois couches (8), la couche centrale (8-2) étant en saillie par rapport aux couches extérieures (8-1, 8-3) et entrant en prise avec ladite au moins une rainure (10).

12. Procédé d'extraction d'un constituant d'un fluide au moyen d'une colonne d'extraction (1) selon la revendication 1, qui comprend un corps de colonne cylindrique orienté verticalement (2), qui forme une cavité de colonne (3), qui présente une extension maximale horizontale (D), et qui est divisée dans la direction verticale en une tête de colonne (A), une zone (B) dans laquelle un dispositif de séparation orienté verticalement (7) est agencé, et un fond de colonne (C), une première entrée (4) étant prévue dans la tête de colonne (A) et une deuxième entrée (5) étant prévue dans le fond de colonne (C), selon le procédé,
le fluide à extraire étant introduit par une des entrées (4, 5) dans la cavité de colonne (3), puis dans plusieurs zones orientées verticalement et séparées horizontalement (B1 à B4 ; B5 à B7 ; B8 à B12), qui sont formées dans la cavité de colonne (3) par le dispositif de séparation orienté verticalement (7), qui divise la cavité de colonne (3) en les zones (B1 à B4 ; B5 à B7 ; B8 à B12), l'extension maximale horizontale (M1 ; M2 ; M3) de chaque zone (B1 à B4 ; B5 à B7 ; B8 à B12) au niveau de chaque coupe horizontale au travers du corps de colonne (2), qui coupe le dispositif de séparation (7), étant inférieure à l'extension maximale horizontale (D) de la cavité de colonne (3), les zones (B1 à B4 ; B5 à B7 ; B8 à B12) débouchant à leurs extrémités supérieures dans la tête de colonne commune (A) et à leurs extrémités inférieures dans le fond de colonne commun (C), et l'extension horizontale de la cavité de colonne (3) n'étant pas divisée par le dispositif de séparation (7) dans la zone de la tête de colonne (A) et dans la zone du fond de colonne (C),
un agent d'extraction étant introduit par l'autre des entrées (4, 5) dans la cavité de colonne (3), puis dans les zones (B1 à B4 ; B5 à B7 ; B8 à B12) de la cavité de colonne (3), et
le constituant du fluide à extraire étant absorbé par l'agent d'extraction dans les zones (B1 à B4 ; B5 à B7 ; B8 à B12) de la cavité de colonne (3) et soutiré en tant que mélange extrait.

13. Procédé selon la revendication 12, **caractérisé en ce que**, dans les zones (B1 à B4 ; B5 à B7 ; B8 à B12), le rapport entre l'agent d'extraction et le fluide à extraire est essentiellement identique sur la section transversale de la colonne d'extraction (1).

14. Procédé selon l'une quelconque des revendications 12 ou 13, **caractérisé en ce que**, dans les zones (B1 à B4 ; B5 à B7 ; B8 à B12), la composition de l'agent d'extraction et/ou la composition du fluide à extraire sont essentiellement identiques sur la section transversale de la colonne d'extraction (1).

15. Procédé selon l'une quelconque des revendications 12 à 14, **caractérisé en ce que** l'agent d'extraction est un solvant organique et le fluide à extraire est de l'eau contenant de l'acide acrylique et de l'acide acétique.
